# EUROPEAN PATENT SPECIFICATION

(11) **EP 4 601 564 B1**
(45) Date of publication and mention of the grant of the patent: **03.06.2026**
(21) Application number: 24752162.8
(22) Date of filing: 15.07.2024
(51) Int. Cl.: A61B 17/32, A61B 17/28, A61B 90/00

(54) **CLAMP ARM OF SURGICAL INSTRUMENT WITH ADJUSTABLE COUPLING STABILITY**
KLEMMARM EINES CHIRURGISCHEN INSTRUMENTS MIT EINSTELLBARER KOPPLUNGSSTABILITÄT
BRAS DE SERRAGE D'INSTRUMENT CHIRURGICAL À STABILITÉ DE COUPLAGE RÉGLABLE

(30) Priority: 17.07.2023 US 202363514011 P; 04.03.2024 US 202418594534
(43) Date of publication of application: 20.08.2025
(73) Proprietor: Cilag GmbH International, 6300 Zug (CH)
(72) Inventor: WORRELL, Barry C., Cincinnati, Ohio 45242 (US); MINNELLI, Patrick J., Cincinnati, Ohio 45242 (US); RUPP, Kip M., Cincinnati, Ohio 45242 (US); RAMANNAIAH, Vasanta Peroor, Bengaluru Karnataka 560024 (IN); DANNAHER, William D., Cincinnati, Ohio 45236 (US); BURKART, Ellen, Cincinnati, Ohio 45040 (US); MANCHEGOWDA, Roopesh Kumar, Mysore Kamataka 560024 (IN)
(74) Representative: Carpmaels & Ransford LLP
(86) International application number: PCT/IB2024/056860
(87) International publication number: WO 2025/017464

(56) References cited:
- EP-A1- 0 214 107
- AU-A1- 2015 258 304
- GB-A- 924 741
- JP-A- 2004 033 565
- US-A- 1 852 542
- US-A- 975 515
- US-A1- 2010 331 873
- US-A1- 2013 303 949
- US-A1- 2015 080 925
- US-A1- 2023 200 839
- US-B2- 10 952 759
- US-B2- 11 020 200
- US-B2- 11 116 532

## Description

### PRIORITY

This application claims priority to U.S. Provisional Pat. App. No. 63/514,011, entitled "Methods of End Effector Assembly and Related Arrangements for Surgical Instruments," filed July 17, 2023.

### BACKGROUND

A variety of surgical instruments include an end effector having a blade element that vibrates at ultrasonic frequencies to cut and/or seal tissue (e.g., by denaturing proteins in tissue cells). These instruments include piezoelectric elements that convert electrical power into ultrasonic vibrations, which are communicated along an acoustic waveguide to the blade element. The precision of cutting and coagulation may be controlled by the surgeon's technique and adjusting the power level, blade edge, tissue traction and blade pressure.

Examples of ultrasonic surgical instruments include the HARMONIC ACE^{®} Ultrasonic Shears, the HARMONIC WAVE^{®} Ultrasonic Shears, the HARMONIC FOCUS^{®} Ultrasonic Shears, and the HARMONIC SYNERGY^{®} Ultrasonic Blades, all by Ethicon Endo-Surgery, Inc. of Cincinnati, Ohio. Further examples of such devices and related concepts are disclosed in U.S. Pat. No. 5,322,055, entitled "Clamp Coagulator/Cutting System for Ultrasonic Surgical Instruments," issued June 21, 1994; U.S. Pat. No. 5,873,873, entitled "Ultrasonic Clamp Coagulator Apparatus Having Improved Clamp Mechanism," issued February 23, 1999; U.S. Pat. No. 5,980,510, entitled "Ultrasonic Clamp Coagulator Apparatus Having Improved Clamp Arm Pivot Mount," filed October 10, 1997; U.S. Pat. No. 6,325,811, entitled "Blades with Functional Balance Asymmetries for use with Ultrasonic Surgical Instruments," issued December 4, 2001; U.S. Pat. No. 6,773,444, entitled "Blades with Functional Balance Asymmetries for Use with Ultrasonic Surgical Instruments," issued August 10, 2004; and U.S. Pat. No. 6,783,524, entitled "Robotic Surgical Tool with Ultrasound Cauterizing and Cutting Instrument," issued August 31, 2004.

Still further examples of ultrasonic surgical instruments are disclosed in U.S. Pub. No. 2006/0079874, entitled "Tissue Pad for Use with an Ultrasonic Surgical Instrument," published April 13, 2006; U.S. Pub. No. 2007/0191713, entitled "Ultrasonic Device for Cutting and Coagulating," published August 16, 2007; U.S. Pub. No. 2007/0282333, entitled "Ultrasonic Waveguide and Blade," published December 6, 2007; U.S. Pub. No. 2008/0200940, entitled "Ultrasonic Device for Cutting and Coagulating," published August 21, 2008; U.S. Pub. No. 2009/0105750, entitled "Ergonomic Surgical Instruments," published April 23, 2009; U.S. Pub. No. 2010/0069940, entitled "Ultrasonic Device for Fingertip Control," published March 18, 2010; and U.S. Pub. No. 2011/0015660, entitled "Rotating Transducer Mount for Ultrasonic Surgical Instruments," published January 20, 2011; and U.S. Pub. No. 2012/0029546, entitled "Ultrasonic Surgical Instrument Blades," published February 2, 2012.

Some of ultrasonic surgical instruments may include a cordless transducer such as that disclosed in U.S. Pub. No. 2012/0112687, entitled "Recharge System for Medical Devices," published May 10, 2012; U.S. Pub. No. 2012/0116265, entitled "Surgical Instrument with Charging Devices," published May 10, 2012; and/or U.S. Pat. App. No. 61/410,603, filed November 5, 2010, entitled "Energy-Based Surgical Instruments," .

Additionally, some ultrasonic surgical instruments may include an articulating shaft section. Examples of such ultrasonic surgical instruments are disclosed in U.S. Pub. No. 2014/0005701, entitled "Surgical Instruments with Articulating Shafts," published January 2, 2014; and U.S. Pub. No. 2014/0114334, entitled "Flexible Harmonic Waveguides/Blades for Surgical Instruments," published April 24, 2014 .

While several surgical instruments and systems have been made and used, it is believed that no one prior to the inventors has made or used the invention described in the appended claims.

US 11 116 532 B2 refers to a surgical instrument comprising an end effector that includes an ultrasonic blade, and a clamp arm that moves relative to the ultrasonic blade from an opened position toward an intermediate position and a closed position. The clamp arm is offset from the ultrasonic blade to define a predetermined gap in the intermediate position between the opened position and the closed position. A clamp arm actuator connects to the clamp arm and moves from an opened configuration to a closed configuration to direct the clamp arm from the opened position toward the intermediate position and the closed position. A spacer connects with the clamp arm to inhibit movement of the clamp arm from the intermediate position toward the closed position for maintaining the predetermined gap between the clamp arm and the ultrasonic blade.

US 2015/080925 A1 refers to an ultrasonic instrument comprising a body, a shaft assembly, an ultrasonic blade, and a pivoting member. The shaft assembly extends distally from the body. The ultrasonic blade is positioned distal to the shaft assembly. The pivoting member is pivotable with respect to the blade from an open position to a closed position to thereby clamp tissue between the pivoting member and the blade. One or both of the shaft assembly or the pivoting member comprise a guide feature. The guide feature is configured to provide lateral alignment of the distal portion of the pivoting member with the blade as the pivoting member is pivoted to the closed position.

US 2013/303949 A1 refers to a forceps type treatment instrument including an ultrasound probe that protrudes from a treatment instrument main body at a position separated to a distal end side from a shaft portion, and a jaw that is displaceable between a closed state and an opened state by being linked with swing action of an clamp arm in a position separated to a distal end side from the shaft portion, stoppers are extendedly provided toward a clamp arm side from a treatment instrument main body side in a state in which edge portions at a distal end side are set to located at a distal end side from a locus of a proximal end of the jaw, and entry of a living body into a gap of the treatment instrument main body and the clamp arm is restricted.

### SUMMARY

The invention is defined by claim 1. Further embodiments of the invention are defined by the dependent claims.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a perspective view of an illustrative surgical instrument;
FIG. 2 depicts a side elevational view of the end effector of the instrument of FIG. 1, in a closed configuration;
FIG. 3 depicts a perspective view of the end effector of FIG. 2, in an open configuration;
FIG. 4 depicts a perspective view of the end effector of FIG. 2, in a closed configuration;
FIG. 5 depicts a perspective view of another illustrative surgical instrument;
FIG. 6 depicts an exploded perspective view of the end effector, the clamp arm assembly, and the shaft assembly of the surgical instrument of FIG. 5,;

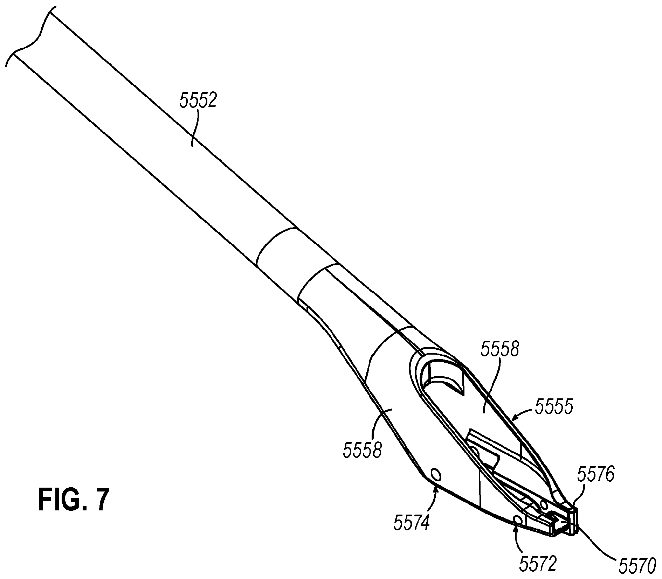

FIG. 7 depicts a perspective view of a shank of the clamp arm assembly of FIG. 6;
FIG. 8 depicts a perspective view of a clamp arm being initially attached to the shank of FIG. 7;
FIG. 9 depicts a perspective view of the assembled clamp arm and the shank of FIG. 8 being initially inserted over the shaft assembly of FIG. 6;
FIG. 10 depicts a perspective view of the assembled end effector of FIG. 6;
FIG. 11 depicts a perspective view of another illustrative surgical instrument;
FIG. 12 depicts an exploded perspective view of the a distal end of the clamp arm assembly of the surgical instrument of FIG. 11;
FIG. 13 depicts perspective view of the underside of the distal end of the clamp arm assembly of FIG. 12;
FIG. 14 depicts a perspective view of a distal end of the shank of the clamp arm assembly of FIG. 12;
FIG. 15 depicts a sectional view of the end effector of the surgical instrument of FIG. 11;
FIG. 16 depicts a cross-sectional view of a pivot mechanism that may be readily incorporated into the surgical instrument of FIGS. 1, 5, or 11;
FIG. 17 depicts a cross-sectional view of a pivot mechanism that may be readily incorporated into the surgical instrument of FIGS. 1, 5, or 11;
FIG. 18 depicts a cross-sectional view of a pivot mechanism that may be readily incorporated into the surgical instrument of FIGS. 1, 5, or 11;
FIG. 19A depicts a cross-sectional view of an illustrative pivot pin having a first axel and a second axel decoupled from each other;
FIG. 19B depicts a cross-sectional view of the pivot pin of FIG. 19A, where the first axel and the second axel are coupled to each other;
FIG. 20 depicts a cross-sectional view of a pivot mechanism that may be readily incorporated into the surgical instrument of FIGS. 1, 5, or 11;
FIG. 21 depicts a perspective view of a pivot mechanism including a pin, a clamp arm, and an outer sheath;
FIG. 22 depicts a cross-sectional view of the seventh pivot mechanism of FIG. 21 including a set screw having a tip;
FIG. 23 depicts a cross-sectional view of the seventh pivot mechanism of FIG. 21 including a set screw having a cup;
FIG. 24 depicts a side view of a first clamp arm having various degrees of compliance;
FIG. 25 depicts a cross-sectional view of segment 25-25 of FIG. 24;
FIG. 26 depicts a side view of a second clamp arm having various degrees of compliance;
FIG. 27 depicts a perspective view of a pivot mechanism;
FIG. 28A depicts a side view of a clamp arm;
FIG. 28B depicts and a sectional view of a portion of the clamp arm of FIG. 28A having a counterbored hole and channel, taken along line 28B-28B of FIG. 28A;
FIG. 29 depicts a cross-sectional view of the fifth pivot mechanism of FIG. 27 with the clamp arm of FIG. 28A;
FIG. 30 depicts a cross-sectional view of the view of FIG. 29 with a mushroomed pin;
FIG. 31 depicts a cross-sectional view of a pivot mechanism including an unflared pin, a clamp arm, and an outer sheath;
FIG. 32 depicts a cross-sectional view of the pivot mechanism of FIG. 31 including a flared pin, a clamp arm, and an outer sheath;
FIG. 33 depicts a side view of a clamp arm assembly including a clamp arm, a thumb grip, a shank, and a sleeve;
FIG. 34 depicts a side view of an ultrasonic surgical instrument having a handle assembly and another clamp arm assembly;
FIG. 35 depicts the ultrasonic surgical instrument of FIG. 34 having a thumb grip ring and clamp arm that may be laser welded together, heat staked, or pinned to form joint;
FIG. 36 depicts the ultrasonic surgical instrument of FIG. 34 having a thumb grip ring and clamp arm that may include a pin along a longitudinal axis and having a configured modulus to thus control an amount of flex;
FIG. 37 depicts the ultrasonic surgical instrument of FIG. 34 having a thumb grip ring that may include a course ball and clamp arm that may include a socket;
FIG. 38 depicts a perspective exploded view of an ultrasonic surgical instrument having a handle assembly and yet another clamp arm assembly;
FIG. 39 depicts a perspective view of a distal tip of the ultrasonic instrument of FIG. 38;
FIG. 40 depicts a bottom view of the distal tip of the ultrasonic instrument of FIG. 39;
FIG. 41 depicts a bottom perspective view of the distal tip of the ultrasonic instrument of FIG. 39;
FIG. 42 depicts a wire-frame bottom perspective view of the distal tip of the ultrasonic instrument of FIG. 39;
FIG. 43 depicts a wire-frame top perspective view of the distal tip of the ultrasonic instrument of FIG. 39;
FIG. 44 depicts a wire-frame top view of the distal tip of the ultrasonic instrument of FIG. 39;
FIG. 45 depicts clamp arm including clamp tip and an adhered clamp pad;
FIG. 46 depicts a perspective view of a clamp arm including a clamp tip, a clamp pad, and a carrier;
FIG. 47 depicts a side view of the clamp arm of FIG. 46;
FIG. 48 depicts a perspective view of the clamp arm of FIG. 46; and
FIG. 49 depicts a perspective cross-sectional view of the clamp arm of FIG. 46.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

For clarity of disclosure, the terms "proximal" and "distal" are defined herein relative to a human or robotic operator of the surgical instrument. The term "proximal" refers the position of an element closer to the human or robotic operator of the surgical instrument and further away from the surgical end effector of the surgical instrument. The term "distal" refers to the position of an element closer to the surgical end effector of the surgical instrument and further away from the human or robotic operator of the surgical instrument.

### I. Illustrative Ultrasonic Surgical Instrument

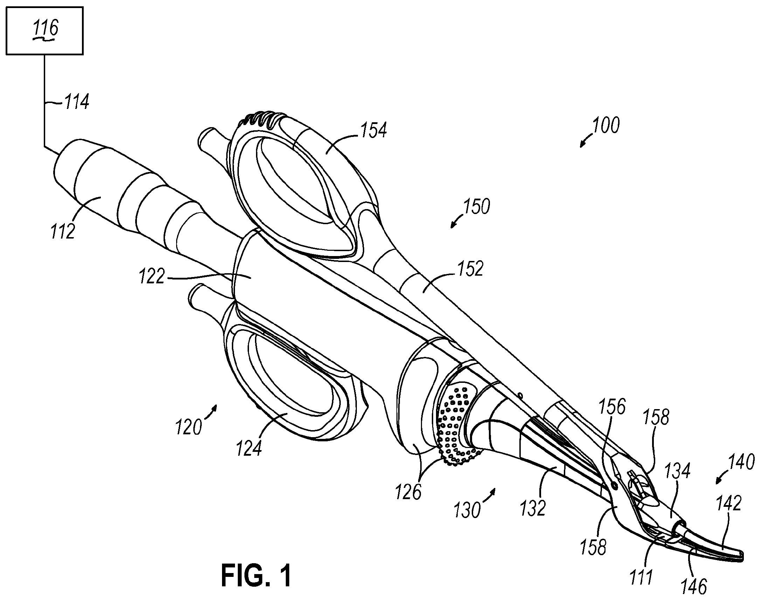

FIGS. 1-4 illustrate an illustrative ultrasonic surgical instrument (100). At least part of each instrument (100) may be constructed and operable in accordance with at least some of the teachings of U.S. Pat. No. 5,322,055; U.S. Pat. No. 5,873,873; U.S. Pat. No. 5,980,510; U.S. Pat. No. 6,325,811; U.S. Pat. No. 6,773,444; U.S. Pat. No. 6,783,524; U.S. Pub. No. 2006/0079874; U.S. Pub. No. 2007/0191713; U.S. Pub. No. 2007/0282333; U.S. Pub. No. 2008/0200940; U.S. Pub. No. 2009/0105750; U.S. Pub. No. 2010/0069940; U.S. Pub. No. 2011/0015660; U.S. Pub. No. 2012/0112687; U.S. Pub. No. 2012/0116265; U.S. Pub. No. 2014/0005701; U.S. Pub. No. 2014/0114334; U.S. Pat. App. No. 61/410,603; and/or U.S. Pat. App. No. 14/028,717. As described therein and as will be described in greater detail below, instrument (100) is operable to cut tissue and seal or weld tissue (e.g., a blood vessel, etc.) substantially simultaneously. It should also be understood that instrument (100) may have various structural and functional similarities with the HARMONIC ACE^{®} Ultrasonic Shears, the HARMONIC WAVE^{®} Ultrasonic Shears, the HARMONIC FOCUS^{®} Ultrasonic Shears, and/or the HARMONIC SYNERGY^{®} Ultrasonic Blades. Furthermore, instrument (100) may have various structural and functional similarities with the devices taught in any of the other references that are cited herein.

To the extent that there is some degree of overlap between the teachings of the references cited herein, the HARMONIC ACE^{®} Ultrasonic Shears, the HARMONIC WAVE^{®} Ultrasonic Shears, the HARMONIC FOCUS^{®} Ultrasonic Shears, and/or the HARMONIC SYNERGY^{®} Ultrasonic Blades, and the following teachings relating to instruments (100), there is no intent for any of the description herein to be presumed as admitted prior art. Several teachings herein will in fact go beyond the scope of the teachings of the references cited herein and the HARMONIC ACE^{®} Ultrasonic Shears, the HARMONIC WAVE^{®} Ultrasonic Shears, the HARMONIC FOCUS^{®} Ultrasonic Shears, and the HARMONIC SYNERGY^{®} Ultrasonic Blades.

FIG. 1 illustrates an illustrative ultrasonic surgical instrument (100) that is configured to be used in open surgical procedures. Instrument (100) of this example comprises a handle assembly (120), a shaft assembly (130), and an end effector (140). Handle assembly (120) comprises a body (122) including a finger grip ring (124) and a pair of buttons (126). Instrument (100) also includes a clamp arm assembly (150) that is pivotable toward and away from body (122). Clamp arm assembly (150) includes a shank (152) with a thumb grip ring (154). Thumb grip ring (154) and finger grip ring (124) together provide a scissor grip type of configuration. It should be understood, however, that various other suitable configurations may be used, including but not limited to a pistol grip configuration.

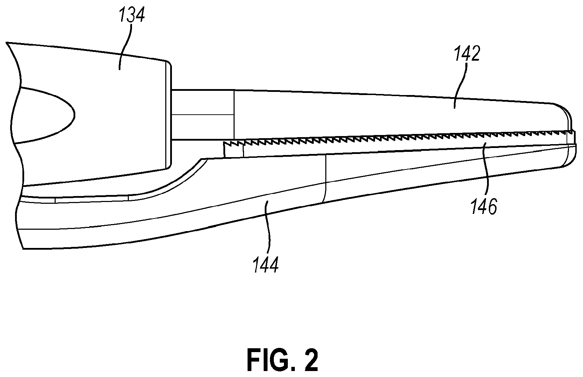

Shaft assembly (130) comprises an outer sheath (132) extending distally from body (122). A cap (134) is secured to the distal end of sheath (132). As best seen in FIGS. 2-4, end effector (140) comprises an ultrasonic blade (142) and a clamp arm (144). Ultrasonic blade (142) extends distally from cap (134). In the current example, clamp arm (144) is an integral feature of clamp arm assembly (150). A distal end of shank (152) terminates into a pair of laterally opposed sidewalls (158), each of which extend distally into clamp arm (144). Laterally opposed sidewalls (158) together define an opening (155) that receives a portion of outer sheath (132).

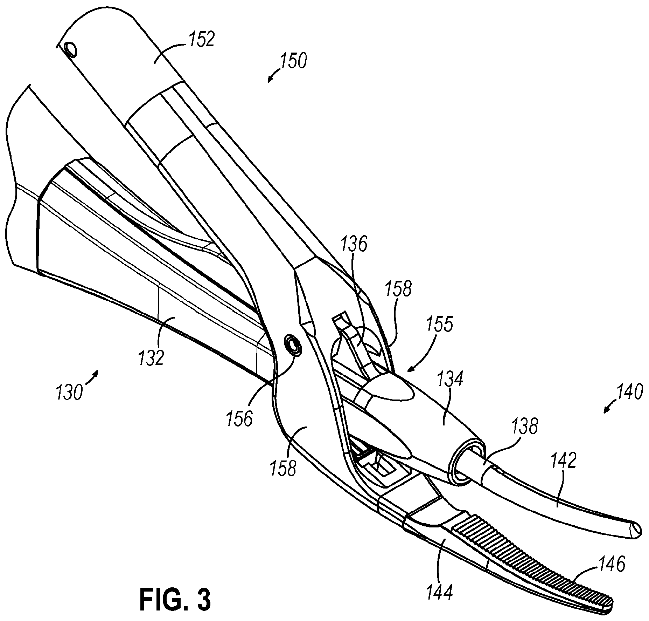

Clamp arm (144) includes a clamp pad (146) facing ultrasonic blade (142). Clamp arm assembly (150) is pivotally coupled with outer sheath (132) via a pin (156). Outer sheath (132) also includes a fin (136) extending upwardly therefrom within the confines of laterally opposed sidewalls (158). Pin (156) extends through suitable portions of laterally opposed sidewall (158) and fin (136) in order to pivotally couple clamp arm assembly (150) with outer sheath (132). Clamp arm (144) is positioned distal to pin (156); while shank (152) and thumb grip ring (154) are positioned proximal to pin (156). Thus, as shown in FIGS. 3-4, clamp arm (144) is pivotable toward and away from ultrasonic blade (142) based on pivoting of thumb grip ring (154) toward and away from body (122) of handle assembly (120). It should therefore be understood that an operator may squeeze thumb grip ring (154) toward body (122) to thereby clamp tissue between clamp pad (146) and ultrasonic blade (142) to transect and/or seal the tissue. In some versions, one or more resilient members are used to bias clamp arm (144) to the open position shown in FIG. 3. By way of example only, such a resilient member may comprise a leaf spring, a torsion spring, and/or any other suitable kind of resilient member.

Referring back to FIG. 1, an ultrasonic transducer assembly (112) extends proximally from body (122) of handle assembly (120). Transducer assembly (112) is coupled with a generator (116) via a cable (114). Transducer assembly (112) receives electrical power from generator (116) and converts that power into ultrasonic vibrations through piezoelectric principles. Generator (116) may include a power source and control module that is configured to provide a power profile to transducer assembly (112) that is particularly suited for the generation of ultrasonic vibrations through transducer assembly (112). By way of example only, generator (116) may comprise a GEN 300 sold by Ethicon Endo-Surgery, Inc. of Cincinnati, Ohio. In addition or in the alternative, generator (116) may be constructed in accordance with at least some of the teachings of U.S. Pub. No. 2011/0087212, entitled "Surgical Generator for Ultrasonic and Electrosurgical Devices," published April 14, 2011. It should also be understood that at least some of the functionality of generator (116) may be integrated into handle assembly (120), and that handle assembly (120) may even include a battery or other on-board power source such that cable (114) is omitted. Still other suitable forms that generator (116) may take, as well as various features and operabilities that generator (116) may provide, will be apparent to those of ordinary skill in the art in view of the teachings herein.

Ultrasonic vibrations that are generated by transducer assembly (112) are communicated along an acoustic waveguide (138), which extends through shaft assembly (130) to reach ultrasonic blade (142). Waveguide (138) is secured within shaft assembly (130) via a pin (not shown), which passes through waveguide (138) and shaft assembly (130). Therefore, waveguide (138) is secured relative to outer sheath (132). This pin is located at a position along the length of waveguide (138) corresponding to a node associated with resonant ultrasonic vibrations communicated through waveguide (138). As noted above, when ultrasonic blade (142) is in an activated state (i.e., vibrating ultrasonically), ultrasonic blade (142) is operable to effectively cut through and seal tissue, particularly when the tissue is being clamped between clamp pad (146) and ultrasonic blade (142). It should be understood that waveguide (138) may be configured to amplify mechanical vibrations transmitted through waveguide (138). Furthermore, waveguide (138) may include features operable to control the gain of the longitudinal vibrations along waveguide (138) and/or features to tune waveguide (138) to the resonant frequency of the system.

In the present example, the distal end of ultrasonic blade (142) is located at a position corresponding to an anti-node associated with resonant ultrasonic vibrations communicated through waveguide (138), in order to tune the acoustic assembly to a preferred resonant frequency fₒ when the acoustic assembly is not loaded by tissue. When transducer assembly (112) is energized, the distal end of ultrasonic blade (142) is configured to move longitudinally in the range of, for example, approximately 10 to 500 microns peak-to-peak, and in some instances in the range of about 20 to about 200 microns at a predetermined vibratory frequency fₒ of, for example, 55.5 kHz. When transducer assembly (112) of the present example is activated, these mechanical oscillations are transmitted through the waveguide to reach ultrasonic blade (142), thereby providing oscillation of ultrasonic blade (142) at the resonant ultrasonic frequency. Thus, when tissue is secured between ultrasonic blade (142) and clamp pad (146), the ultrasonic oscillation of ultrasonic blade (142) may simultaneously sever the tissue and denature the proteins in adjacent tissue cells, thereby providing a coagulative effect with relatively little thermal spread. In some versions, an electrical current may also be provided through ultrasonic blade (142) and/or clamp pad (146) to also seal the tissue.

An operator may activate buttons (126) to selectively activate transducer assembly (112) to thereby activate ultrasonic blade (142). In the present example, two buttons (126) are provided - one for activating ultrasonic blade (142) at a low power and another for activating ultrasonic blade (142) at a high power. However, it should be understood that any other suitable number of buttons and/or otherwise selectable power levels may be provided. For instance, a foot pedal may be provided to selectively activate transducer assembly (112). Buttons (126) of the present example are positioned such that an operator may readily fully operate instrument (100) with a single hand. For instance, the operator may position their thumb in thumb grip ring (154), position their ring finger in finger grip ring (124), position their middle finger about body (122), and manipulate buttons (126) using their index finger. Of course, any other suitable techniques may be used to grip and operate instrument (100); and buttons (126) may be located at any other suitable positions.

The foregoing components and operabilities of instrument (100) are merely illustrative. Instrument (100) may be configured in numerous other ways as will be apparent to those of ordinary skill in the art in view of the teachings herein. By way of example only, at least part of instrument (100) may be constructed and/or operable in accordance with at least some of the teachings of any of the following: U.S. Pat. No. 5,322,055; U.S. Pat. No. 5,873,873; U.S. Pat. No. 5,980,510; U.S. Pat. No. 6,325,811; U.S. Pat. No. 6,783,524; U.S. Pub. No. 2006/0079874; U.S. Pub. No. 2007/0191713; U.S. Pub. No. 2007/0282333; U.S. Pub. No. 2008/0200940; U.S. Pub. No. 2010/0069940; U.S. Pub. No. 2011/0015660; U.S. Pub. No. 2012/0112687; U.S. Pub. No. 2012/0116265; U.S. Pub. No. 2014/0005701; U.S. Pub. No. 2014/0114334; and/or U.S. Pat. App. No. 14/031,665. Additional merely illustrative variations for instrument (100) will be described in greater detail below. It should be understood that the below described variations may be readily applied to instrument (100) described above and any of the instruments referred to in any of the references that are cited herein, among others

### II. Ultrasonic Surgical Instrument with Alignment Features for Ultrasonic Blade and Clamp Pad

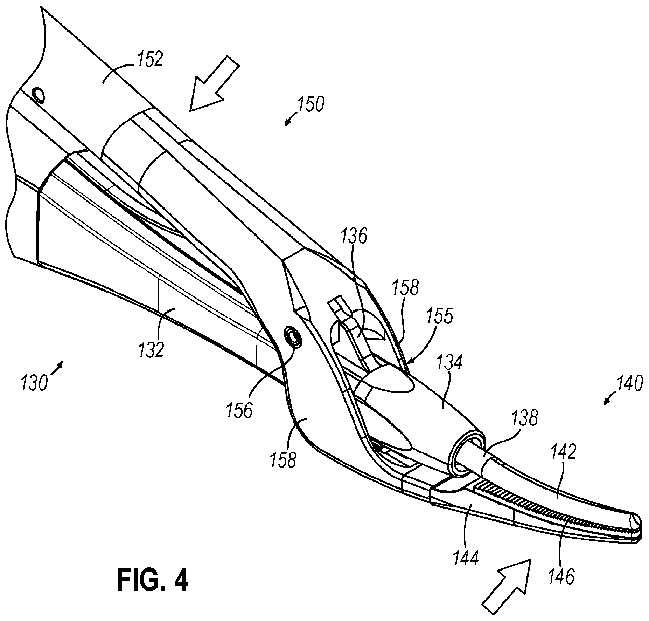

As mentioned above, clamp arm (144) is pivotally coupled to ultrasonic blade (142) between an open position (*see* FIG. 3) and a closed position (*see* FIG. 4) in order to suitably grasp tissue between clamp pad (146) and ultrasonic blade (142), and then activate ultrasonic blade (142) in order to transect and/or seal the grasped tissue. As also mentioned above, in the current example, clamp arm (144) is integrally formed with shank (152) of clamp arm assembly (150). In some instances, it may be desirable for clamp arm (144) and shank (152) to be formed of different materials. For instance, thermal energy may accumulate at end effector (140) during illustrative use, such that a metal material may be more suitable for clamp arm (144) to inhibit undesirable deformation in response to exposure to accumulated thermal energy. However, it may be desirable to utilize a material having different properties for shank (152), such as a lighter weight material (e.g., a suitable plastic). Of course, clamp arm (144) and shank (152) may be formed of any suitable first material and second material, respectively, as would be apparent to one skilled in the art in view of the teachings herein.

In some instances when clamp arm (144) and shank (152) are formed of different materials and/or components, clamp arm (144) and shank (152) may require being coupled together during assembly of instrument (100). During assembly of instrument (100), an undesirable tolerance stack may accumulate. For example, a tolerance stack may accumulate when clamp arm (144) and shank (152) are assembled together. As another example, a tolerance stack may accumulate when shank (152) and shaft assembly (130) are pivotally coupled together. Such tolerance stacks may undesirably affect the alignment between clamp pad (146) and ultrasonic blade (142) when assembled, which may lead to undesirable effects of sealed and/or welded tissue. For example, in some instances, a user may desire to grasp tissue with just a distal tip of ultrasonic blade (142) and clamp pad (146) (otherwise known as "tip nibling"), thereby transecting and/or sealing smaller portions of tissue, rather than sections of tissue that coincide along a substantial length of clamp pad (146) and/or ultrasonic blade (142). Grasping tissue with the ultrasonic blade (142) and clamp pad (146) via "tip nibling" may allow a user to control the areas of tissue which are transected and/or sealed with greater precision. However, in instances where ultrasonic blade (142) and clamp pad (146) fail to suitably align, the longitudinal ends of ultrasonic blade (142) and clamp pad (146) may also fail to suitably align with each other, which may undesirably affect the quality of a tissue transection and/or sealing via tip nibbling. Therefore, it may be desirable to have features to promote suitable alignment between ultrasonic blade (146) and clamp arm (144)/clamp pad (146) when end effector (140) is suitably assembled and ready for illustrative use, especially in instances where clamp arm (144) and shank (152) are separate components that are coupled together during assembly of instrument (100).

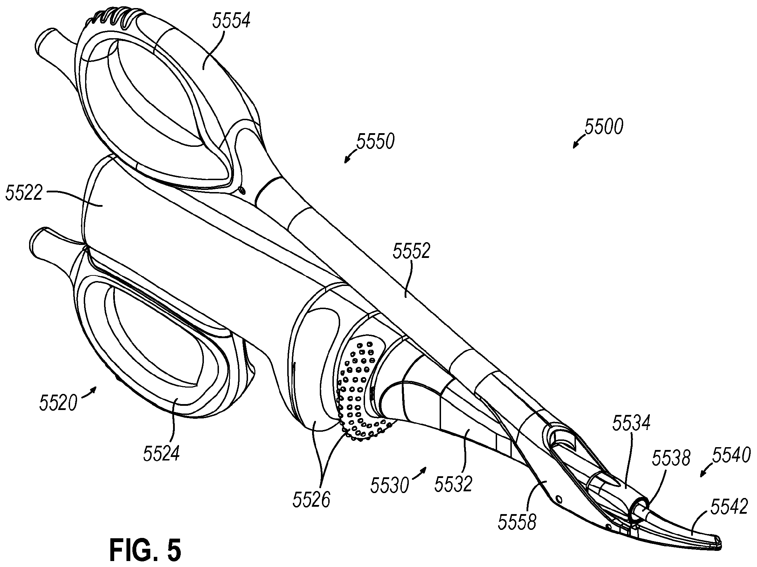

FIG. 5 shows an illustrative ultrasonic surgical instrument (5500) that may be used in replacement of ultrasonic surgical instrument (100) described above. Therefore, ultrasonic surgical instrument (5500) is substantially similar to instrument (100) described above, with differences elaborated herein. Instrument (5500) includes a handle assembly (5520), a shaft assembly (5530), an end effector (5540), and a clamp arm assembly (5550); which may be substantially similar to handle (120), shaft assembly (130), end effector (140), and clamp arm assembly (150), respectively, with differences elaborated herein.

As will be described in greater detail below, a clamp arm (5544) of end effector (5540) and shank (5552) of clamp arm assembly (5550) are separate components that are coupled together during assembly of instrument (5500). As will also be described in greater detail below, clamp arm (5544) is configured to pivotally couple directly to outer sheath (5532), thereby reducing an accumulated tolerance stack and further promoting proper alignment (longitudinal and/or lateral) of clamp arm (5554)/clamp pad (5546) with ultrasonic blade (5542).

Handle assembly (5520) includes a body (5522), a finger grip ring (5524), and buttons (5526); which may be substantially similar to body (122), finger grip ring (124), and buttons (126) described above. While not shown, it should be understood that body (5522) may suitably couple to ultrasonic transducer assembly (112) (similar to body (122) described above) such that ultrasonic transducer assembly (112) may suitably communicate ultrasonic vibrations to ultrasonic waveguide (5538) and ultrasonic blade (5542) in accordance with the description above. Shaft assembly (5530) includes an outer sheath (5532), a cap (5534), and an ultrasonic waveguide (5538); which may be substantially similar to sheath (132), cap (134), and ultrasonic waveguide (138) described above, with differences elaborated below. Sheath (5532) of the current example includes a pivotal coupling body (5536). Unlike fin (136) of sheath (132) described above, pivotal coupling body (5536) extends away from outer sheath (5532) in a downward direction extending away from a proximal portion of shank (5552). As will be described in greater detail below, the location of pivotal coupling body (5536) allows clamp arm (5544) to pivotally couple directly with outer sheath (5532).

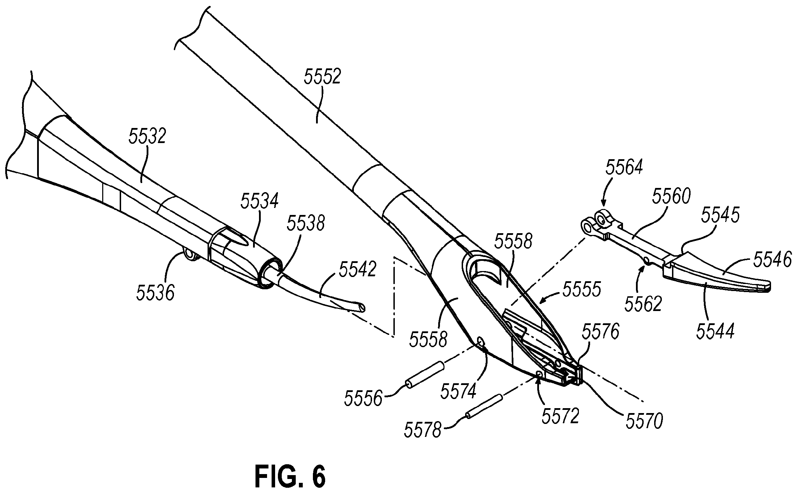

Clamp arm assembly (5550) includes a shank (5552) and a thumb ring grip (5554); which may be substantially similar to shank (152) and thumb ring grip (154) described above, with differences elaborated herein. Shank (5552) and thumb ring grip (5554) may be formed of suitable material, such as a plastic material. Turning to FIG. 6, a distal end of shank (5552) includes a pair of laterally opposed sidewalls (5558) defining a cavity (5555). Laterally opposed sidewalls (5558) and cavity (5555) may be substantially similar to laterally opposed sidewalls (158) and cavity (155) described above, with differences elaborated herein. Therefore, when instrument (5500) is assembled, cavity (5555) is dimensioned to suitably house a portion of outer sheath (5532).

At least one laterally opposed sidewall (5558) defines a clamp arm coupling through hole (5572) and a pivot through hole (5574). As will be described in greater detail below, clamp arm coupling through hole (5572) is configured to receive a pin (5578) in order to suitably attach clamp arm (5544) with shank (5552) such that pivoting of shank (5552) relative to outer sheath (5532) drives pivoting of clamp arm (5544) relative to ultrasonic bade (5542). As will also be described in greater detail below, pivot through hole (5574) is dimensioned to receive pivot pin (5556) in order to allow pivot pin (5556) to directly pivotally couple clamp arm (5544) with pivotal coupling body (5536) of outer sheath (5532) via a sheath coupling through hole (5564) defined by clamp arm (5544). Directly pivotally coupling clamp arm (5544) with outer sheath (5532) may reduce an undesirable tolerance stack as compared to coupling clamp arm (5544) directly to shank (5552), which is then pivotally coupled to outer sheath (5532). Reducing this undesirable tolerance stack allows clamp arm (5544), and therefore clamp pad (5546), to be better aligned with ultrasonic blade (5542) once instrument (5500) is assembled.

A distal end of shank (5552) also includes a clamp arm support surface (5570) extending between laterally oppose sidewalls (5558) and also defining a portion of cavity (5555). When shank (5552) is pivotally coupled to outer sheath (5532) in accordance with the description herein, clamp arm support surface (5570) is located adjacent to the underside of outer sheath (5532). In other words, clamp arm support surface (5570) is located adjacent to a portion of outer sheath (5532) associated with pivotal coupling body (5536). Clamp arm support surface (5570) is dimensioned to receive and support an elongated coupling body (5560) of clamp arm (5544). Therefore, a proximal end of clamp arm (5544) may suitably align with clamp arm support surface (5570), and then be proximally inserted into the confines of clamp arm support surface (5570) until through holes (5572, 5574) defined by at least one laterally opposed sidewall (5558) are suitably aligned to a corresponding through hole (5562, 5564) defined by clamp arm (5544). In conjunction with pin (5578), clamp arm support surface (5570) may include suitable complementary geometry to respective portions of elongated coupling body (5560) of clamp arm (5544) in order to operatively retain clamp arm (5544) relative to shank (5552), thereby inhibiting undesirable relative movement between clamp arm (5540) and shank (5552) during illustrative use in accordance with the teachings herein.

A distal end of shank (5552) also includes a clamp pad stop (5576). In the current example, clamp arm (5544) is configured to selectively couple with clamp pad (5546). In particular, clamp arm (5544) and clamp pad (5546) may couple together via complementary geometries (e.g., a tongue and groove relationship) such that clamp pad (5546) may slide distally along a predetermined path defined by the complementary mating features of clamp arm (5544) and clamp pad (5546) in order to initially couple with each other. Once clamp pad (5546) is suitably coupled to clamp arm (554), and once clamp arm (5544) is suitably coupled to shank (5554), clamp pad stop (5576) is directly adjacent to a proximal surface (5545) of clamp pad (5546). Clamp pad stop (5576) is configured to directly engage a proximal surface (5545) of clamp pad (5546) in order to inhibit clamp pad (5546) from sliding proximally out of alignment relative to clamp arm (5554). Therefore, clamp pad stop (5576) is configured to maintain longitudinal alignment of clamp pad (5546) relative to clamp arm (5544) once clamp arm (5544) and clamp pad (5546) are suitably coupled with shank (5552) in accordance with the description herein.

As mentioned above, clamp arm (5544) includes an elongated coupling body (5560) defining a shank coupling through hole (5562) and a sheath coupling through hole (5564). Clamp arm (5544) may be formed of suitable second material, such as a metallic material. Therefore, in some instances, shank (5552) may be formed of a first material having desirable characteristics for shank (5552) (e.g., a plastic material), while clamp arm (5544) may be formed of a second material having desirable characteristics for clamp arm (5544) (e.g., a metallic material). Shank coupling through hole (5562) is dimensioned to align with clamp arm coupling through hole (5572) of shank (5552) once elongated coupling body (5560) of clamp arm (5544) is suitably inserted into clamp arm support surfacer (5570). Once through holes (5562, 5572) are aligned, pin (5578) is inserted into both through holes (5562, 5572) in order to suitably couple clamp arm (5544) with shank (5552) in accordance with the description herein.

Sheath coupling through hole (5564) is dimensioned to align with pivot through hole (5574) of shank (5552) once elongated coupled body (5560) of clamp arm (5544) is suitably inserted into clamp arm support surface (5570). As mentioned above, pivot pin (5556) is configured to directly pivotally couple outer sheath (5532) with clamp arm (5544) via pivotal coupling body (5536) and sheath coupling through hole (5564) in order to reduce an accumulated tolerance stack during assembly of instrument (5500) to thereby promote suitable alignment between clamp arm (5544)/clamp pad (5546) with ultrasonic blade (5542) during illustrative use of instrument (5500) in accordance with the description herein. Therefore, sheath coupling through hold (5564), pivot pin (5556), and pivotal coupling body (5536) act as a clamp arm alignment structure that promotes proper alignment of clamp arm (5544)/clamp pad (5545) with ultrasonic blade (5542) while clamp arm (5544) is in a closed position.

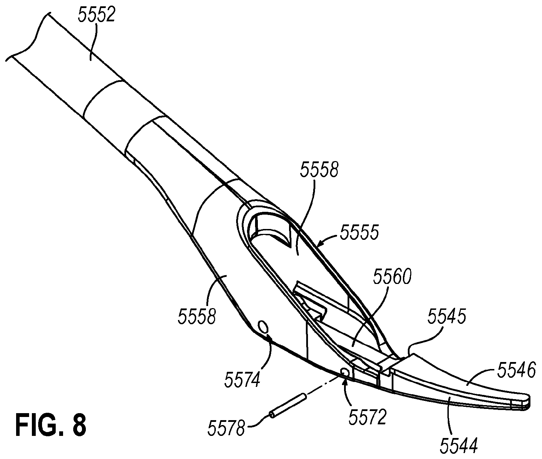

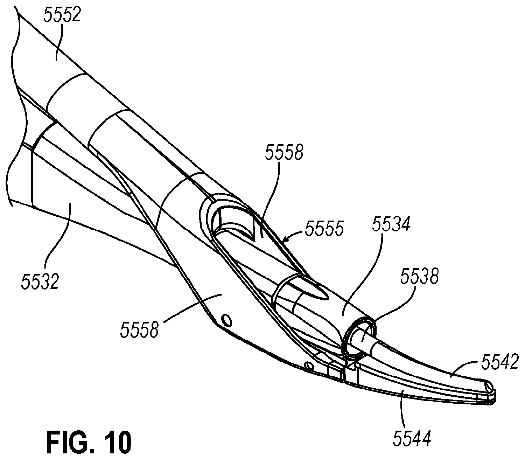

FIGS. 8-10 show an illustrative assembly of clamp arm (5540), shank (5552), and shaft assembly (5530). First, as shown in FIG. 8, clamp arm (5544) may be initially inserted within the confines of cavity (5555) such that elongated coupling body (5560) of clamp arm (5544) is suitably housed within clamp arm support surface (5570) of shank (5552). Once suitably inserted, through holes (5562, 5564) of elongated coupling body (5560) are suitably aligned with respective through holes (5572, 5574) of shank (5552). Once through holes (5562, 5572) are suitably aligned, pin (5578) is inserted into both through holes (5562, 5572), thereby attaching clamp arm (5540) to shank (5542) such that movement of shank (5552) drives corresponding movement of clamp arm (5540). It should also be understood that in instances where clamp pad stop (5576) is incorporated, clamp pad (5546) is inhibited from disassociating with clamp arm (5544) via engagement between clamp pad stop (5576) and proximal facing surface (5548) of clamp arm (5546).

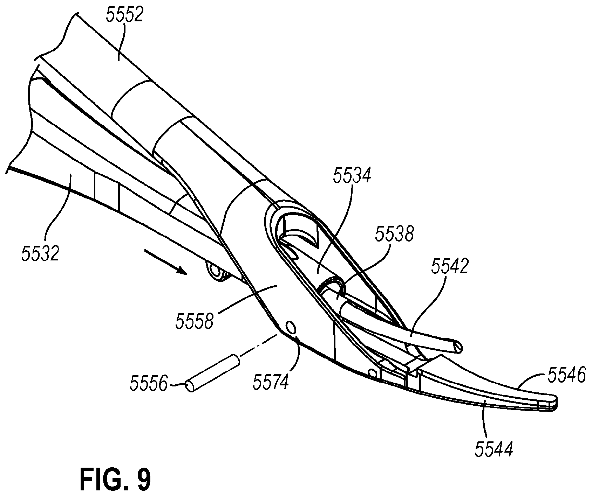

Next, as shown in FIG. 9, ultrasonic blade (5542) may be inserted through cavity (5555) defined by laterally opposed sidewalls (5558) and clamp arm support surface (5570) until sheath coupling through hole (5564) of clamp arm (5552) and pivot through hole (5574) of shank (5552) are both aligned with pivotal coupling body (5536) of outer sheath (5532). Next, pivot pin (5556) is inserted through pivot through hole (5574) of shank (5552), sheath coupling through hole (5564) of clamp arm (5552), and pivotal coupling body (5536) of sheath (5532) in order to pivotally couple shank (5552) and clamp arm (5544) with outer sheath (5532) in accordance with the description herein. Once pivotally coupled, shank (5552) and clamp arm (5544) may pivot relative to sheath (5532) and ultrasonic blade (5542) in order to suitably grasp tissue in accordance with the description herein. It should be understood that pin (5556) is dimensioned to suitably engage sheath coupling through hold (5564) of clamp arm (5544) and pivotal coupling body (5536) of outer sheath (5532) to thereby promote alignment between clamp arm (5544) with ultrasonic blade (5542) by reducing the accumulated tolerance stack during the assembly of instrument (5500). The location of pivotally coupling body (5536) allows clamp arm (5544) to directly pivotally couple with outer sheath (5532) without having to make further modifications to clamp arm (5544).

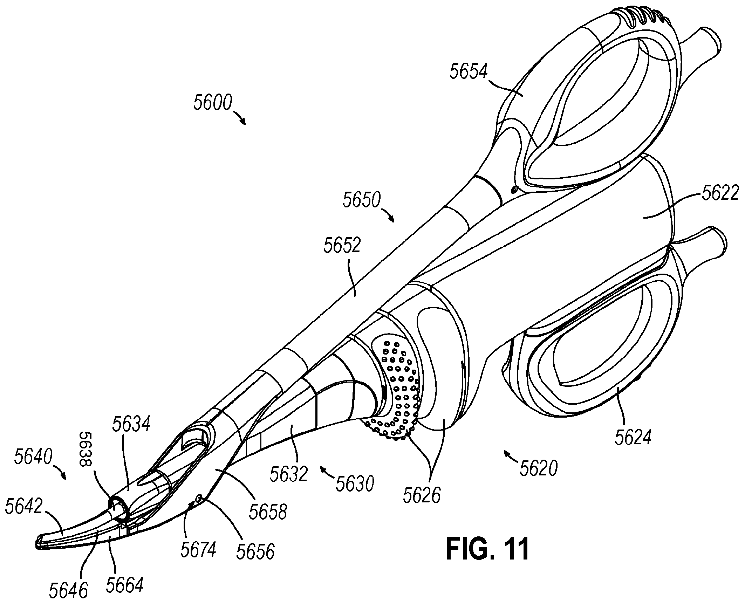

FIG. 11 shows an illustrative ultrasonic surgical instrument (5600), in accordance with the present claimed invention, that may be used in replacement of ultrasonic surgical instrument (100) described above. Therefore, ultrasonic surgical instrument (5600) is substantially similar to instrument (100) described above, with differences elaborated herein. Instrument (5600) includes a handle assembly (5620), a shaft assembly (5630), an end effector (5640), and a clamp arm assembly (5650); which may be substantially similar to handle (120), shaft assembly (130), end effector (140), and clamp arm assembly (150), respectively, with differences elaborated herein.

As will be described in greater detail below, a clamp arm (5644) of end effector (5640) and shank (5652) of clamp arm assembly (5650) are separate components that are coupled together during assembly of instrument (5600). As will also be described in greater detail below, clamp arm (5644) is configured to be adjustable relative to shank (5652) prior to being affixed relative to shank (5652), thereby allowing clamp arm (5644) to suitably align with ultrasonic blade (5642) after shank (5652) pivotally couples to an outer sheath (5632) of shaft assembly (5630). Providing customizable adjustability of clamp arm (5644) relative to shank (5652) after shank (5652) is pivotally coupled to shaft assembly (5630) reduces an accumulated tolerance stack and further promotes alignment (longitudinal and/or lateral) of clamp arm (5644)/clamp pad (5646) with ultrasonic blade (5642).

Handle assembly (5620) includes a body (5622), a finger grip ring (5624), and buttons (5626); which may be substantially similar to body (122), finger grip ring (124), and buttons (126) described above. While not shown, it should be understood that body (5622) may suitably couple to ultrasonic transducer assembly (112) (similar to body (122) described above) such that ultrasonic transducer assembly (112) may suitably communicate ultrasonic vibrations to ultrasonic waveguide (5638) and ultrasonic blade (5642) in accordance with the description above. Shaft assembly (5630) includes an outer sheath (5632), a cap (5634), and a ultrasonic waveguide (5638); which may be substantially similar to sheath (132), cap (134), and ultrasonic waveguide (138) described above, with differences elaborated below. Sheath (5632) of the current example includes a pivotal coupling (not shown) that may be substantially similar to pivotal coupling body (5536) described above. Therefore, unlike fin (136) of sheath (132) described above, pivotal coupling (not shown) extends away from outer sheath (5632) in a downward direction extending away from a proximal portion of shank (5652).

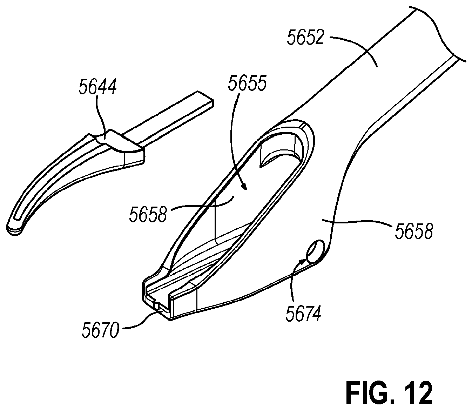

Clamp arm assembly (5650) includes a shank (5652) and a thumb ring grip (5654); which may be substantially similar to shank (152) and thumb ring grip (154) described above, with differences elaborated herein. Shank (5652) and thumb ring grip (5654) may be formed of suitable material, such as a plastic material. Turning to FIG. 12, a distal end of shank (5652) includes a pair of laterally opposed sidewalls (5658) defining a cavity (5655). Laterally opposed sidewalls (5658) and cavity (5655) may be substantially similar to laterally opposed sidewalls (158) and cavity (155) described above, with differences elaborated herein. Therefore, when instrument (5600) is assembled, cavity (5655) is dimensioned to suitably house a portion of outer sheath (5632). At least one side wall (5558) defines a through hole (5674) dimensioned receive pivot pin (5656) to pivotally couple shank (5652) to outer sheath (5632).

Clamp arm (5644) includes an elongated coupling body (5660). Clamp arm (5644) may be formed of suitable second material, such as a metallic material. Therefore, in some instances, shank (5652) may be formed of a first material having desirable characteristics for shank (5652) (e.g., a plastic material), while clamp arm (5644) may be formed of a second material having desirable characteristics for clamp arm (5644) (e.g., a metallic material). As will be described in greater detail below, clamp arm coupling body (5660) is dimensioned to fit within a clamp arm cavity (5686) defined by a clamp arm mounting support (570) of shank (5652).

A distal end of shank (5652) also includes a clamp arm mounting support (5570) extending between laterally oppose sidewalls (5658) and also defining a portion of cavity (5655). When shank (5652) is pivotally coupled to outer sheath (5632) in accordance with the description herein, clamp arm mounting support (5670) is located adjacent to the underside of outer sheath (5632). In other words, clamp arm mounting support (5670) is located adjacent to a portion of outer sheath (5632) associated with pivotal coupling (not shown).

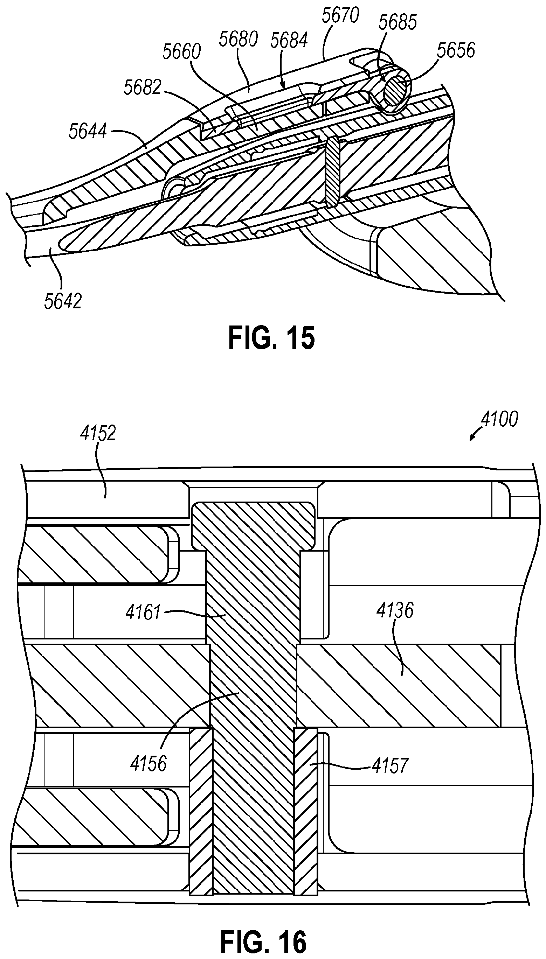

Clamp arm mounting support (5670) includes a base (5680) and a welding plate (5682). In some instances, welding plate (5682) may be fixed and secured to base (5680), such as via an over-molding process. Turning to FIG. 15, welding plate (5682) defines a pivot opening (5685) dimensioned to receive pivot pin (5656). Therefore, welding plate (5782) is configured to directly pivotally couple to outer sheath (5632). Base (5680) may be formed of the same material as shank (5652). Therefore, in the current example, base (5680) is formed of a plastic material. Welding plate (5682) is formed of a suitable material configured to be secured to elongated coupling body (5660) of clamp arm (5644) via welding, such as a metallic material.

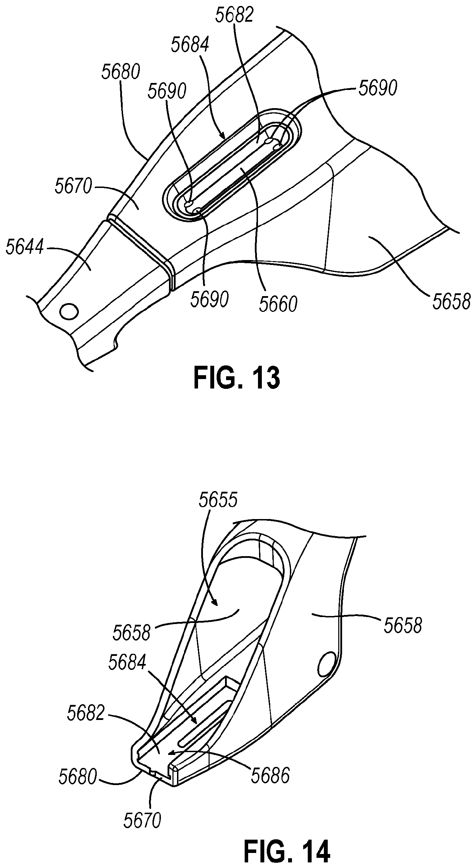

Clamp arm mounting support (5670) defines clamp arm cavity (5686) that is dimensioned to receive an elongated coupling body (5660) of clamp arm (5644). As best shown in FIGS. 13-15, welding plate (5682) and base (5680) define a welding window (5684). When elongated coupling body (5660) of clamp arm (5644) is suitably inserted into clamp arm cavity (5685), a portion of elongate coupling body (5660) is accessible via welding window (5684).

Clamp arm mounting support (5670) is dimensioned to receive and support an elongated coupling body (5660) of clamp arm (5644). During assembly, a proximal end of clamp arm (5644) may suitably align with clamp arm mounting support (5670), and then be proximally inserted into the confines of clamp arm cavity (5686). Prior to being welded to welding plate (5682) in accordance with the description herein, elongated coupling body (5660) is adjustable within clamp arm cavity (5686) relative to clamp arm mounting support (5670) such that clamp arm (5644) may be positioned into suitable alignment with ultrasonic blade (5642). Prior to welding clamp arm (5644) to welding plate (5682), clamp arm (5644) may be positioned relative to clamp arm mounting support (5570) via rotation about one or more suitable axes, translation along one or more suitable paths, and/or a combination of rotation and translation along any suitable path was would be apparent to one skilled in the art in view of teachings herein. Once clamp arm (5644) is positioned into suitable alignment with ultrasonic blade (5642), elongated coupling body (5560) is then permanently secured relative to clamp arm support (5670) (e.g., via welding to metal plate of clamp arm support (5670)). It should be understood that access for securing elongated coupling body (5560) to clamp arm support (5670) is provided via welding window (5684).

Therefore, during assembly, welding window (5684), welding plate (5682), and elongated coupling body (5660) are configured to reduce an accumulated tolerance stack during assembly of instrument (5600) to thereby promote suitable alignment between clamp arm (5644)/clamp pad (5646) with ultrasonic blade (5642) during illustrative use of instrument (5600) in accordance with the description herein. Welding window (5684), welding plate (5682), and elongated coupling body (5666) act as a clamp arm alignment structure to promote proper alignment of clamp arm (5644)/clamp pad (5645) with ultrasonic blade (5642) while clamp arm (5644) is in a closed position.

### III. Clamp Arm of Surgical Instrument with Adjustable Coupling Stability and Related Methods

It may be of benefit to include an adjustable compression pivot mechanism between clamp arm assembly (150) and shaft assembly (130), thereby enabling selectable friction at the pivot point of pin (156). By increasing the compression, the frictional resistance of pivoting clamp arm assembly (150) relative to shaft assembly (130) at the pivot point of pin (156) can be increased. By reducing the compression, the frictional resistance of pivoting clamp arm assembly (150) relative to shaft assembly (130) at the pivot point of pin (156) can be decreased. Manufacturer set compression allows each assembly to be custom adjusted to a specific compression utilizing the friction coefficient irrespective of the tolerance combinations on the clamp arm and pivot shaft. Controlling the pivotal frictional resistance between clamp arm assembly (150) and shaft assembly (130) may provide the user with real time mechanical feedback during illustrative use of instrument (100) that improves the perception of device. Detailed below are examples of such pivot mechanisms that may be incorporated in whole or in part into one or more of surgical instruments, such as surgical instrument (100), described herein.

FIG. 16 depicts a cross-sectional view of a pivot mechanism (4100) that may be readily incorporated into instrument (100, 5500, 5600) described above. Pivot mechanism (4100) includes a shank (4152), an outer sheath (4132) having a fin (4136), and a pin (4156); which may be readily incorporated into instrument (100) in replacement of shank (152), outer sheath (132), fin (136), and pin (156) described above, respectively. Therefore, shank (4252), outer sheath (4232), fin (4236), and pin (4256) may be substantially similar to shank (152), outer sheath (132), fin (136), and pin (156) described above, respectively, with differences elaborated herein.

Pin (4156) pivotally couples shank (4152) with outer sheath (4132). Pin (4156) includes a shoulder (4161) configured to compress against a side surface of either shank (4152) or fin (4136) of outer sheath (4132). Pivot mechanism (4100) further includes a bushing (4157) configured to be inserted over the end of pin (4156) opposite of first shoulder (4161) during assembly. In particular, when shank (4152) and outer sheath (4132) are already pivotally coupled via pin (4156), bushing (4157) is configured to be inserted over pin (4156) toward shoulder (4161) such that bushing (4157) engages the side surface of shank (4152) or fin (4136) that is not engaged with shoulder (4161) of pin (4156). In other words, bushing (4157) is inserted over pin (4156) such that bushing (4157) and shoulder (4161) compress surfaces of fin (4136) and shank (4152) toward each other, thereby customizing the frictional resistance of pivoting shank (4152) relative to outer sheath (4132).

Once a suitable frictional resistance is created based on the spacing of bushing (4157) relative to pin (4156), bushing (4157) may be welded to pin (4156) in order to control the pivotal fictional resistance between shank (4152) and outer sheath (4132). Additionally, the dimension/placement of bushing (4157) may be selected in order suitably align clamp pad (146) with ultrasonic blade (142) in accordance with the teachings herein during assembly. In other words, the outer radius of bushing (4157) and or radial position of bushing (4157) relative to pin (4156) may be selected in order to suitably space shank (4152) relative to outer sheath (4132) such that clamp pad (146) (which is attached to shank (4152)) and ultrasonic blade (142) (which is suitably attached to outer sheath (4132)) are suitably aligned.

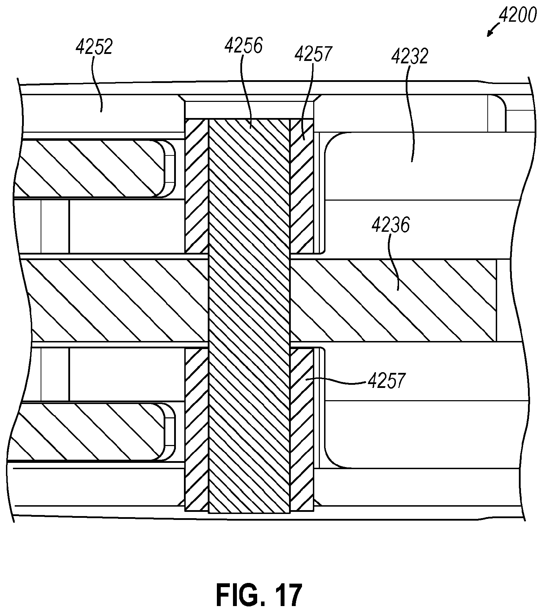

FIG. 17 depicts a cross-sectional view of another pivot mechanism (4200) that may be readily incorporated into instrument (100, 5500, 5600) described above. Pivot mechanism (4200) includes a shank (4252), an outer sheath (4232) having a fin (4236), and a pin (4256); which may be readily incorporated into instrument (100) in replacement of shank (152), outer sheath (132), fin (136), and pin (156) described above, respectively. Therefore, shank (4252), outer sheath (4232), fin (4236), and pin (4256) may be substantially similar to shank (152), outer sheath (132), fin (136), and pin (156) described above, respectively, with differences elaborated herein.

Pivot mechanism (4200) in the current example includes two bushings (4257) located on opposite ends of pin (4156). Together, bushings (4257) function in substantially similar manner as shoulder (4161) and bushing (4157) described above. Therefore, after shank (4252) and outer sheath (4232) are pivotally coupled via pin (4156), each bushing (4257) maybe advanced along opposite ends of pin (4256) in order to compress portions of outer sheath (4232) and shank (4252) toward each other, thereby customizing the frictional resistance of pivoting shank (4252) relative to outer sheath (4232). Once a suitable frictional resistance is created based on the spacing of bushings (4257) relative to pin (4256), shank (4252), and outer sheath (4232), bushings (4257) may be welded to pin (4256) in order to control the pivotal fictional resistance between shank (4252) and outer sheath (4232). Additionally, the dimension/placement of bushings (4257) may be selected in order suitably align clamp pad (146) with ultrasonic blade (142) in accordance with the teachings herein during assembly. In other words, the outer radius of bushings (4257) and or radial position of bushing (4257) relative to pin (4256) may be selected in order to suitably space shank (4252) relative to outer sheath (4232) such that clamp pad (146) (which is attached to shank (4252)) and ultrasonic blade (142) (which is suitably attached to outer sheath (4232)) are suitably aligned.

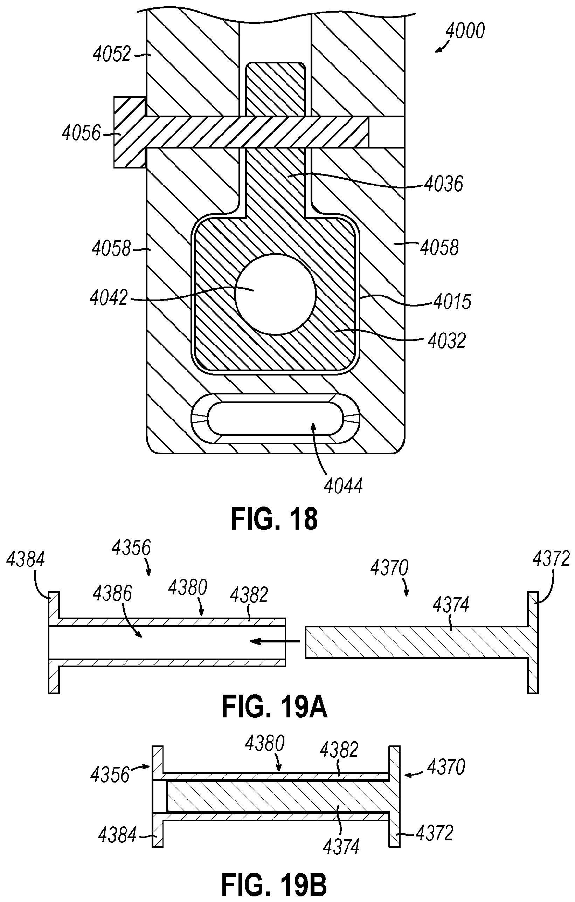

FIG. 18 depicts another pivot mechanism (4000) that may be readily incorporated into instrument (100, 5500, 5600) described above. Pivot mechanism (4000) includes a shank (4052) having sidewalls (4058), clamp arm (4044), an outer sheath (4032) having a fin (4036), and a pin (4056); which may be readily incorporated into instrument (100) in replacement of shank (152), sidewalls (158), clamp arm (144), outer sheath (132), fin (136), and pin (156) described above, respectively. Therefore, shank (4052), clamp arm (4044), outer sheath (4032), fin (4036), and pin (4056) may be substantially similar to shank (152), clamp arm (144) outer sheath (132), fin (136), and pin (156) described above, respectively, with differences elaborated herein.

Shank (4052) is configured to pivot relative to outer sheath (4032). Laterally opposed side walls (4058) include an interior surface (4015) that is directly adjacent to respective sidewalls of outer sheath (4032). Outer sheath (4032) and laterally opposed sidewalls (4058) may be of dissimilar materials with different wear characteristics, such as metal and plastic, respectively. Outer sheath (4032) may be configured to fit within the confines of laterally opposed side walls (4058) such that there is an interference fit along the contacting surfaces (4015) of laterally opposed sidewalls (4058) and the respective sidewall of outer sheath (4032). The interference fit may be "low level" such that shank (4052) may still pivot relative to outer sheath (4032). Since the outer sheath (4032) and the opposed sidewalls (4058) are made of materials with different wear characteristics, the frictional resistance created by the "low level" interference between the laterally opposed sidewalls (4058) and outer sheath (4032) may slightly remove portions of opposed sidewalls (5058) in response to repeatedly and rapidly pivoting shank (4052) relative to outer sheath (4032). Removal of portion of opposed sidewalls (5058) may then reduce the frictional resistance to pivoting shank (4052) relative to outer sheath (4032), effectively tuning (e.g., controlling the level of) frictional resistant of pivoting shank (4052) relative to outer sheath (4032).

The interference fit may remove any lateral gap between opposed sidewalls (4058) and outer sheath (4032) such that there may be no lateral movement between the two components to thus result in increased alignment between clamp pad (146) and ultrasonic blade (142).

Additional mechanisms for tuning include creating clearance with the exception of a small radial crush rib (not shown) on the inside of contacting surfaces (4015) or measuring and binning various outer sheath (4032) widths relative to the plastic components.

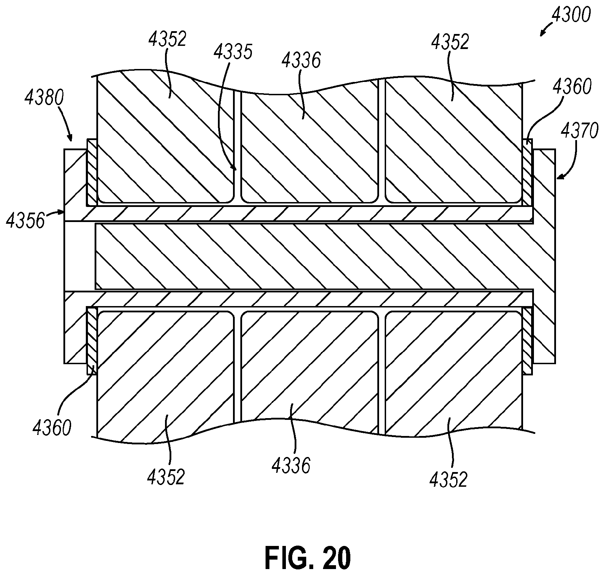

FIG. 20 depicts a cross-sectional view of another pivot mechanism (4300) may be readily incorporated into instrument (100, 5500, 5600) described above, while FIGS. 19A-19B depict a cross-sectional view of a pin (4356) of pivot mechanism (4300). Pivot mechanism (4300) includes a shank (4352), an outer sheath having a fin (4336), and a pin (4356); which may be readily incorporated into instrument (100) in replacement of shank (152), outer sheath (132), fin (136), and pin (156) described above, respectively. Therefore, shank (4352), fin (4336), and pin (4356) may be substantially similar to shank (152), fin (136), and pin (156) described above, respectively, with differences elaborated herein.

As best shown in FIGS. 19A-19B, pin (4356) includes complementary axles (4370, 4380) that are configured to mate with each other in order to adjust the overall length of pin (4356). First axel (4370) includes an inner elongated member (4374) dimensioned to fit within a complementary opening (4286) defined by an outer elongated member (4384) of second axel (4380). Inner elongated member (4374) may be suitably positioned within the confines of complementary opening(4286) such that inner elongated member (4374) and outer elongated member (4384) cooperatively allow for the overall length of pin (4356) to be adjusted (prior to being fixed together in accordance with the description herein). Each axel (4370, 4380) also includes a flange (4374, 4384). Flanges (4374, 4384) impart a compressive force on fin (4336) and shank (4352) such that adjustment of the length of pin (4356) may suitably adjust the frictional resistance to pivoting shank (4352) relative to fine (4336) of outer sheath.

During assembly, axles (4370, 4380) are inserted into the aligned pin openings (4335) denied by fin (4336) of outer sheath and shank (4352) at opposite ends such that inner elongated member (4374) is suitably housed within outer elongated member (4384). The overall length of pin (4356) is adjusted such that flange (4374, 4384) impart the desired amount of compressive force between fin (4336) and shank (4352), thereby creating the desired amount of frictional pivotal resistance. Once the desired amount of compressive force is generated, axles (4370, 4380) are welded together, thereby affixing the distance between axels (4370, 4380). In some instance, bearing washers (4360) are interposed between a flange (4374, 4384) and shank (4352) to ensure the proper amount of frictional pivotal resistance is generated.

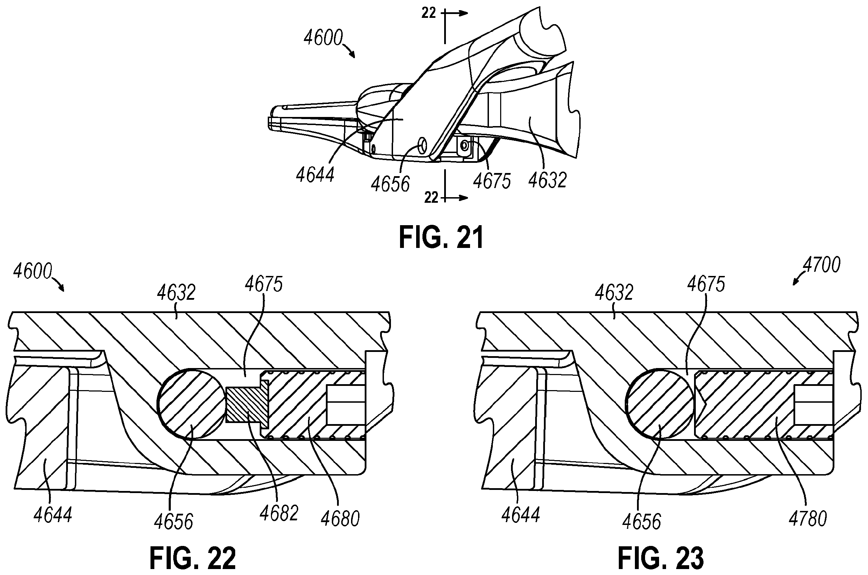

FIGS. 21-22 depicts another pivot mechanism (4600) may be readily incorporated into instrument (100, 5500, 5600) described above. including a pin (4656), a shank (4644), and an outer sheath (4632). Pivot mechanism (4600) includes a shank (4644), an outer sheath (4632), and a pin (4656); which may be readily incorporated into instrument (100) in replacement of shank (152), outer sheath (132), and pin (156) described above, respectively. Therefore, shank (4652), outer sheath (4632), and pin (656) may be substantially similar to shank (152), outer sheath (132), and pin (156) described above, respectively, with differences elaborated herein.

Outer sheath (4632) includes a threaded opening (4765) in communication with pin (4656). Pin (4656) may be secured, such as welded or adhered, at opposing ends to shank (4644). As shown in FIG. 22, a set screw (4680) may be threaded into threaded opening (4675) to thereby apply a force against pin (4656). The force applied to pin (4656) by set screw (4680) may apply a frictional force against pin (4656). The force applied by set screw (4680) may be selectively adjusted by either advancing or retracting set screw (4680) within threaded opening (4765). With set screw (44680) threadably attached to outer sheath (4632) via threaded opening (4675), and pin (4656) secured to shank (4644), the frictional force generated by engagement between set screw (4680) and pin (4656) adjusts the frictional resistance to pivoting shank (4644) relative to outer sheath (4632). Therefore, the frictional resistance to pivoting shank (4644) relative to outer sheath (4632) may be adjusted to a desired amount. Set screw (4680) may include a tip (4682) of a different material to remaining portions of set screw (4680). Tip (4682) may comprise nylon.

FIG. 23 depicts an alternative set screw (4780) having a cup shape nearest the pin (4656) configured to apply a friction force against pin (4656).

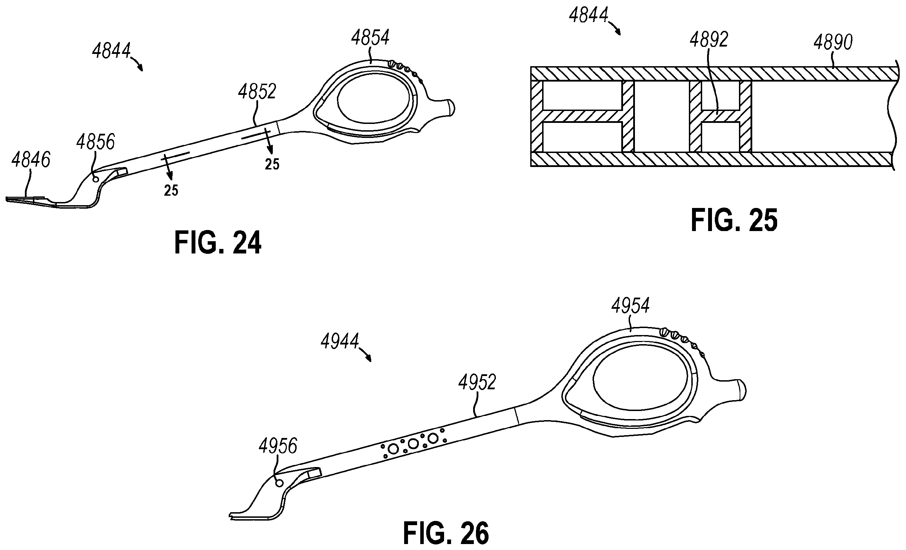

FIG. 24 depicts an alternative clamp arm assembly (4844) that may be readily incorporated into instrument (100, 5500, 5600) described above in replacement of clamp arm assembly (150, 5550, 5650). Therefore, clamp arm assembly (4844) may be substantially similar to clamp arm assembly (150, 5550, 5650) described above, with differences elaborated herein. In particular, clamp arm assembly (4844) has variations in compliance along the length of shank (4852). In other words, some longitudinal sections of shank (4852) may be stiffer (i.e., less prone to elastic deflection in response to grasping tissue in accordance with the teachings herein), while other longitudinal sections may be more compliant (i.e., more prone to elastic deflection in response to grasping tissue in accordance with the teachings herein.). Control the variations of complaint along the length of shank (4852) may allow for a controlled deflection profile during illustrative use in accordance with the description herein.

Clamp arm assembly (4844) may include a thumb grip ring (4854), a clamp tip (4846), a shank (4852), and a pin hole (4856). In order to achieve variable compliance along shank (4852) when an operator applies a force against thumb grip ring (4854), shank (4852) may include regions of varying compliance. FIG. 25 depicts a cross-sectional view of segment A-A of FIG. 24. Region (4890) with no reinforcement ribs may be a region of high compliance, region (4892) with tightly packed ribs may be a region of low compliance, and region (4894) with spaced apart ribs may be a region of medium compliance relative to regions (4890, 4892).

FIG. 26 depicts a side view of a second clamp arm assembly (4944) that is substantially similar to clamp arm assembly (4844) described above, with difference elaborated herein. Clamp arm (4944) includes a thumb grip ring (4954), a shank (4952), and a pin hole (4956), which may be substantially similar to thumb grip ring (5854), shank (4852), and pin hole (4856) described above, with differences elaborated below. In particular, rather than having ribs dictate the level of compliance having various degrees of compliance, shank (4952) is porous along desired longitudinal sections such that a more porous region may be more compliant compared to a less porous region. Porosity may vary along shank (4952).

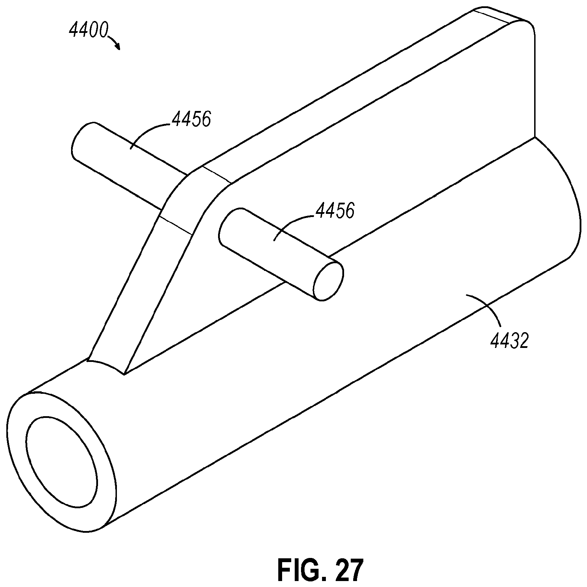

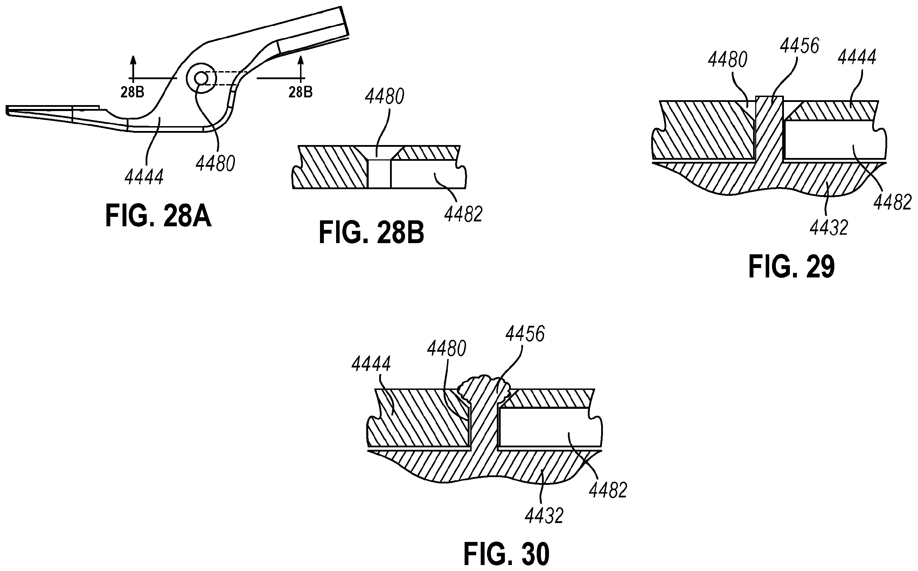

FIG. 27 depicts a perspective view of a fifth pivot mechanism (4400) including outer sheath (4432) having pin (4456). Pin (4456) may be integral to outer sheath (4432) upon initial production of outer sheath (4432) or may be included after and may include a radius between a sidewall of outer sheath (4432) and pin (4456). FIG. 28A depicts a side view of clamp arm (4444) and FIG. 28B depicts a sectional view (Section A) of a portion of clamp arm (4444) having a counterbored hole (4480) and channel (4482). Channel (4482) may be configured to allow pin (4456) to transition towards axial alignment with counterbored hole (4480) during assembly. Once pin (4456) and counterbored hole (4480) are axially aligned, as shown in FIG. 29, lateral ends of pin (4456) may be heated or pressed to thus mushroom in counterbore portion of counterbored hole (4480) as shown in FIG. 30.

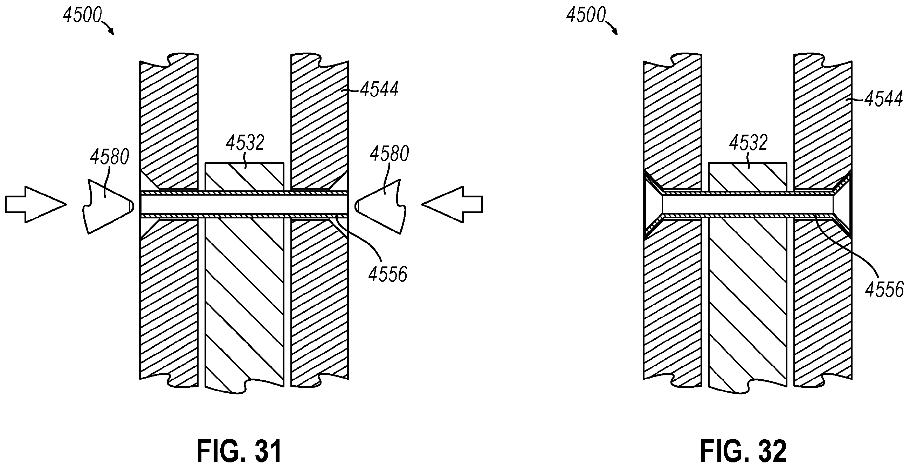

FIG. 31-32 show cross-sectional views of a sixth pivot mechanism (4500) including a pin (4556), a clamp arm (4544), and an outer sheath (4532). As shown in FIG. 31, pin (4556) may have opposing cone mandrels (4580) directed towards a bore of pin (4556) to thus flare ends of pin (4556). By flare ends of pin (4556), pin (4556) may be secured to clamp arm (4544). Pin (4556) may also be press fit into outer sheath (4532) to thus provide a friction fit between outer sheath (4532) and clamp arm (4544). Cone mandrels (4580) may be hollow and may be positioned into a hollow bore of pin (4556) as indicated by arrows. FIG. 32 depicts pin (4556) in the flared configuration.

In an alternative embodiment (not shown) a partially slotted pin may be included instead of pin (4556). Cone mandrels (4580) may be changed to wedge shaped pieces rather than cones to flare the ends of the pin.

In another alternative embodiment (not shown) a hollow tube geometry or slotted pin geometry may be presented on one side of a flanged pin.

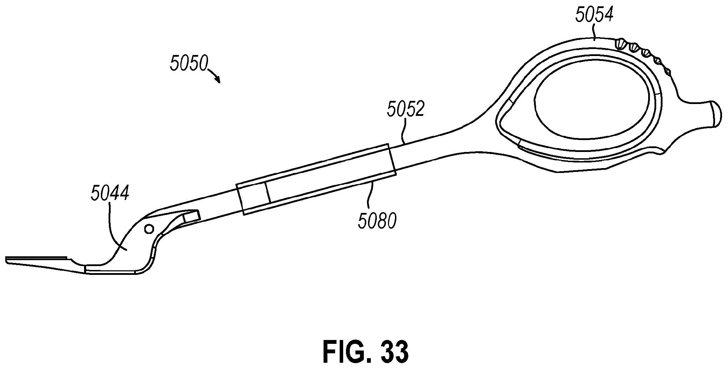

FIG. 33 depicts a side view of a clamp arm assembly (5050) including a clamp arm (5044), a thumb grip (5054), a shank (5052), and a sleeve (5080). Clamp arm (5044) and thumb grip (5054) may be made from different materials and processes. Sleeve (5052) may be used to couple clamp arm (5044) and thumb grip (5054) along shank (5052). Sleeve (5052) may be a cylindrical tube that will harden and/or shrink around clamp arm (5044) and thumb grip (5054) to thereby couple the two. Sleeve (5052) may also be made of metal and be adhered to thumb grip (5054) and clamp arm (5044) to thereby couple the two.

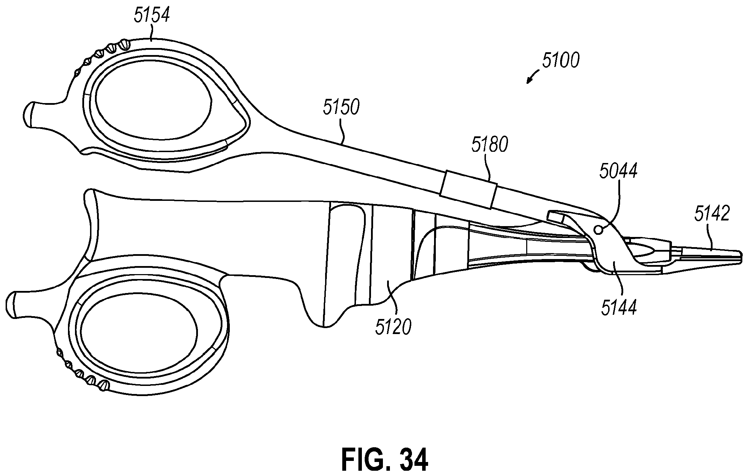

FIG. 34 depicts a side view of an ultrasonic surgical instrument (5100) having a handle assembly (5120) and another clamp arm assembly (5150). Handle assembly (5120) may include an outer sheath (5132) and ultrasonic blade (5142). Clamp arm assembly (5150) may include clamp arm (5144), thumb grip ring (5154), pin (5156), and joint (5180). Joint (5180) may be adjustable to change an angle (θ) between thumb grip ring (5154) and handle assembly (5120).

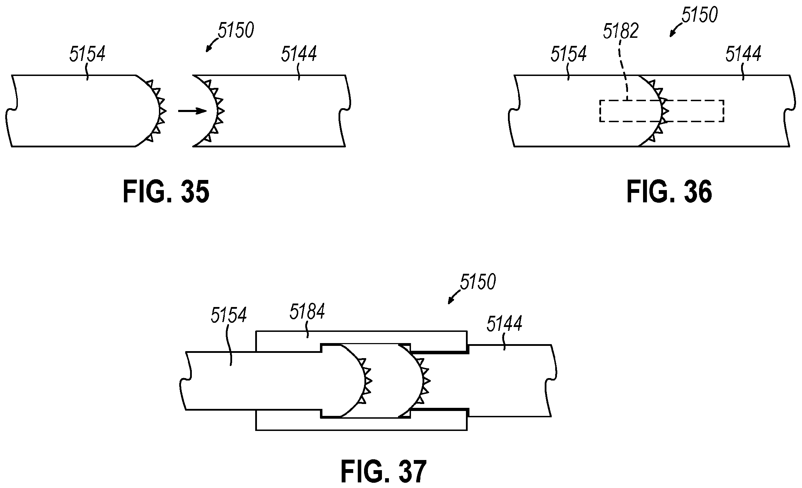

As shown in FIG 35, thumb grip ring (5154) and clamp arm (5144) may be laser welded together, heat staked, or pinned to form joint (5180). As shown in FIG. 36, thumb grip ring (5154) and clamp arm (5144) may include pin (5182) along a longitudinal axis and having a configured modulus to thus control an amount of flex between thumb grip ring (5154) and clamp arm (5144). As shown in FIG. 37, thumb grip ring (5154) may include a course ball and clamp arm (5144) may include a socket. Metal ring (5184) may be welded to either or both thumb grip ring (5154) and clamp arm (5144).

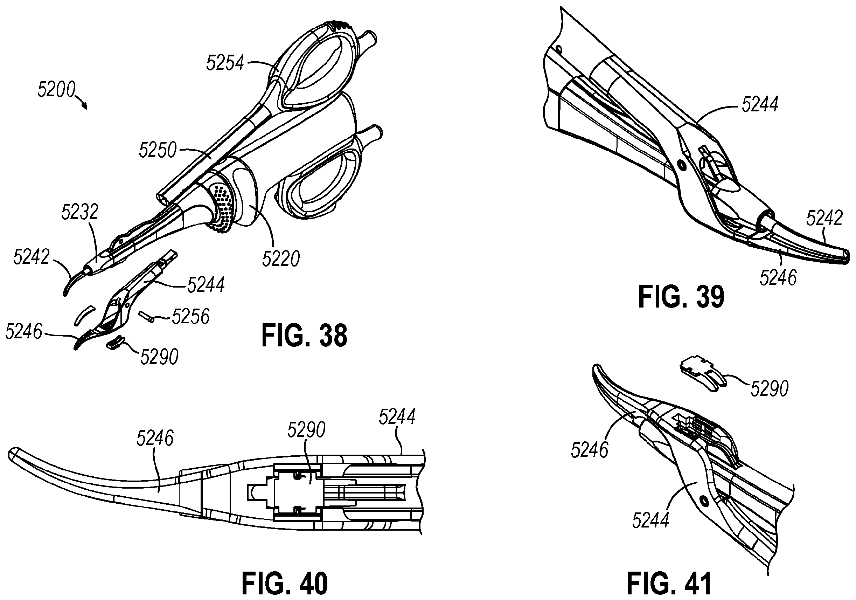

FIG. 38 depicts a perspective exploded view of ultrasonic surgical instrument (5200) having a handle assembly (5220) and yet another clamp arm assembly (5250). Handle assembly (5220) may include an outer sheath (5232) and ultrasonic blade (5242). Clamp arm assembly (5250) may include clamp arm (5244), thumb grip (5254), and pin (5256). Pin (5256) may be an orbital rivet configured to apply frictional forces to clamp arm (5244) and outer sheath (5232). Clamp arm (5244) may be metal injection molded (MIM) and may be assembled or overmolded to thumb grip (5254). Clamp tip (5246) may be MIM which may be a 2 shot MIM with a constant radius key slot configured to fit a clamp pad.

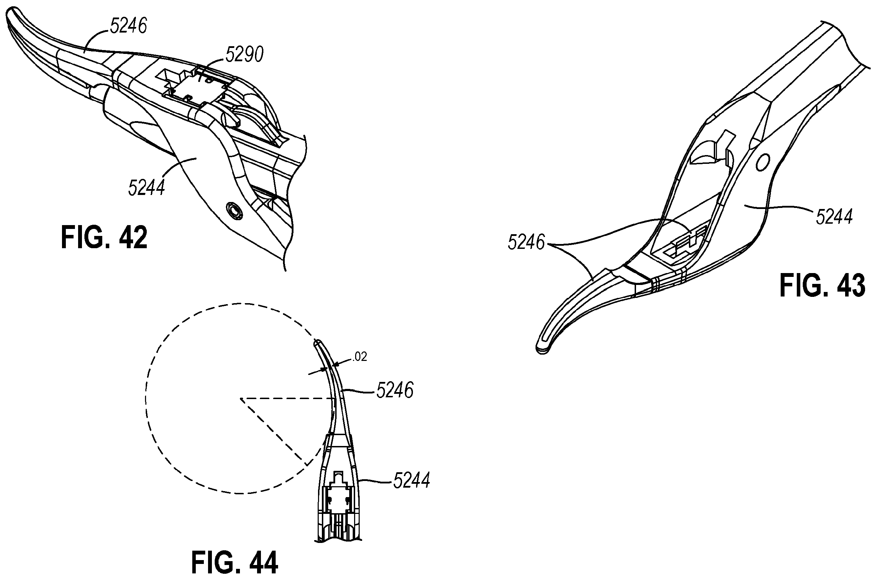

As shown in FIGS. 38-44, clamp tip (5246) may be affixed to clamp arm (5244) by sliding proximally protruding ends of clamp tip (5246) into a cavity of clamp arm (5244). During assembly, ultrasonic blade (5242) may be aligned with clamp tip (5246) and then cap (5290) may be welded to clamp arm (5244) to secure/lock clamp tip (5246) in place for consistent alignment.

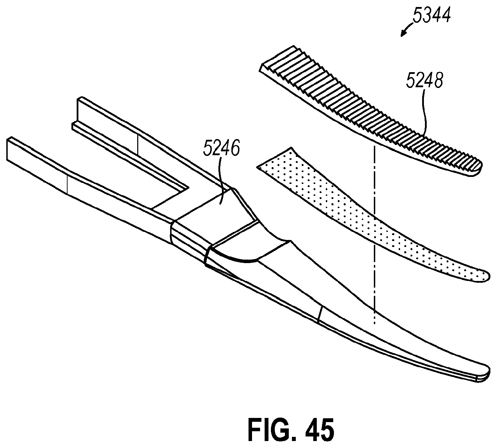

FIG. 45 depicts clamp arm (5344) including clamp tip (5346) and clamp pad (5348). Clamp tip (5346) may be metal, such as stainless steel, and clamp pad (5348) may be bonded to clamp tip (5346) using a PTFE pre-treatment and adhesives. A pre-treatment may be applied and within the application window the adhesive would be applied following an appropriate curing time. Pre-treatment may include a sodium ammonia etch, a plasma etch, and a PTFE primer.

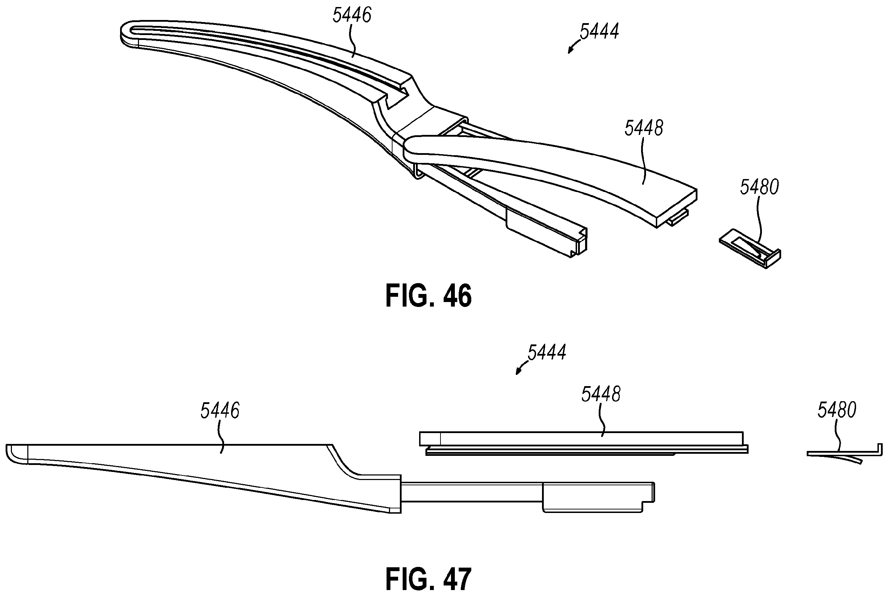

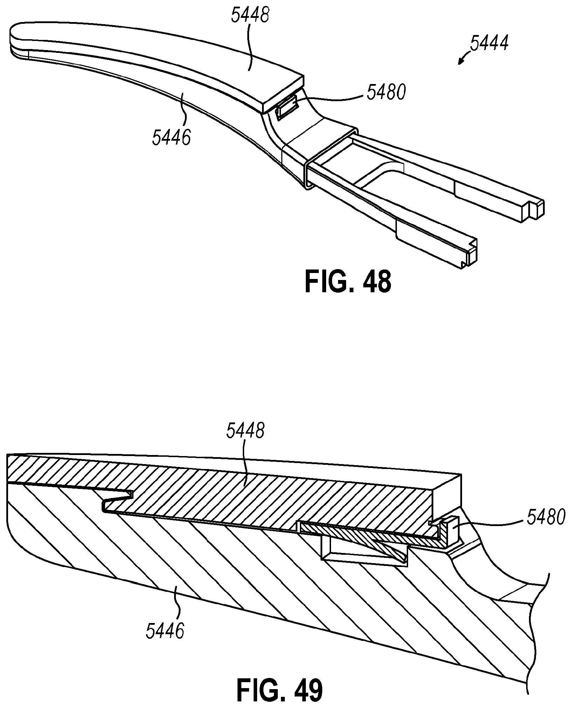

FIGS. 46-49 show a clamp arm (5444) including a clamp tip (5446), a clamp pad (5448), and a carrier (5480). Clamp tip (5446) may include T-Slot configured to receive clamp pad (5448). Carrier (5480) may be a stamped metal and may include a spring configured to lock clamp pad (5448) into clamp tip (5446) to thus prevent clamp pad (5448) from backing out. Carrier (5480) may also be welded to clamp tip (5446) to thus prevent clamp pad (5448) from backing out.

### V. Miscellaneous

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of any claims.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI^{™} system by Intuitive Surgical, Inc., of Sunnyvale, California. Similarly, those of ordinary skill in the art will recognize that various teachings herein may be readily combined with various teachings of any of the following: U.S. Pat. No. 5,792,135, entitled "Articulated Surgical Instrument For Performing Minimally Invasive Surgery With Enhanced Dexterity and Sensitivity," issued Aug. 11, 1998; U.S. Pat. No. 5,817,084, entitled "Remote Center Positioning Device with Flexible Drive," issued Oct. 6, 1998; U.S. Pat. No. 5,878,193, entitled "Automated Endoscope System for Optimal Positioning," issued March 2, 1999; U.S. Pat. No. 6,231,565, entitled "Robotic Arm DLUS for Performing Surgical Tasks," issued May 15, 2001; U.S. Pat. No. 6,783,524, entitled "Robotic Surgical Tool with Ultrasound Cauterizing and Cutting Instrument," issued Aug. 31, 2004; U.S. Pat. No. 6,364,888, entitled "Alignment of Master and Slave in a Minimally Invasive Surgical Apparatus," issued April 2, 2002; U.S. Pat. No. 7,524,320, entitled "Mechanical Actuator Interface System for Robotic Surgical Tools," issued April 28, 2009; U.S. Pat. No. 7,691,098, entitled "Platform Link Wrist Mechanism," issued April 6, 2010; U.S. Pat. No. 7,806,891, entitled "Repositioning and Reorientation of Master/Slave Relationship in Minimally Invasive Telesurgery," issued Oct. 5, 2010; U.S. Pat. No. 8,844,789, entitled "Automated End Effector Component Reloading System for Use with a Robotic System," issued Sept. 30, 2014; U.S. Pat. No. 8,820,605, entitled "Robotically-Controlled Surgical Instruments," issued Sept. 2, 2014; U.S. Pat. No. 8,616,431, entitled "Shiftable Drive Interface for Robotically-Controlled Surgical Tool," issued Dec. 31, 2013; U.S. Pat. No. 8,573,461, entitled "Surgical Stapling Instruments with Cam-Driven Staple Deployment Arrangements," issued Nov. 5, 2013; U.S. Pat. No. 8,602,288, entitled "Robotically-Controlled Motorized Surgical End Effector System with Rotary Actuated Closure Systems Having Variable Actuation Speeds," issued Dec. 10, 2013; U.S. Pat. No. 9,301,759, entitled "Robotically-Controlled Surgical Instrument with Selectively Articulatable End Effector," issued April 5, 2016; U.S. Pat. No. 8,783,541, entitled "Robotically-Controlled Surgical End Effector System," issued July 22, 2014; U.S. Pat. No. 8,479,969, entitled "Drive Interface for Operably Coupling a Manipulatable Surgical Tool to a Robot," issued July 9, 2013; U.S. Pat. Pub. No. 8,800,838, entitled "Robotically-Controlled Cable-Based Surgical End Effectors," issued Aug. 12, 2014; and/or U.S. Pat. No. 8,573,465, entitled "Robotically-Controlled Surgical End Effector System with Rotary Actuated Closure Systems," issued Nov. 5, 2013.

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a clinician immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention is understood not to be limited to the details of structure and operation shown and described in the specification and drawings.

The invention is defined by the following claims.

## Claims

1. A surgical instrument (5600), comprising:
(a) an end effector (5640), the end effector comprising:
(i) an ultrasonic blade (5642), and
(ii) a clamp arm (5644) configured to pivot relative to the ultrasonic blade between an open position and a closed position, wherein the clamp arm comprises an elongated coupling body (5660);
(b) a shaft assembly (5630), the shaft assembly comprising:
(i) an outer sheath (5632), and
(ii) an ultrasonic waveguide (5638) secured relative to outer sheath, wherein the ultrasonic waveguide is at least partially housed within the outer sheath;
(c) a shank (5652) attached to the clamp arm, wherein the shank is configured to pivot relative to the shaft assembly in order to drive the clamp arm between the open position and the closed position, wherein the shank comprises a clamp arm support (5670) configured to receive the elongated coupling body of the clamp arm in order to assist attachment of the clamp arm to the shank; and
(d) a clamp arm alignment structure configured to promote alignment of the clamp arm relative to the ultrasonic blade in the closed position, **characterized in that** the clamp arm alignment structure comprises a welding plate (5682) associated with the clamp arm support of the shank.

2. The surgical instrument of claim 1, wherein the welding plate is configured to receive the elongated coupling body of the clamp arm.

3. The surgical instrument of claim 2, wherein the welding plate defines a welding window (5684) such that the elongated coupling body of the clamp arm is configured to be secured to the welding plate after being initially inserted into the clamp arm support.

4. The surgical instrument of claim 3, wherein the elongated coupling body is configured to be adjusted relative to the welding plate prior to being secured to the welding plate.

5. The surgical instrument of claim 4, wherein the welding plate is directly pivotally coupled to the outer sheath via a pin (5656).

6. The surgical instrument of claim 1, further comprising a handle assembly (5620), wherein the shaft assembly extends distally from the handle assembly.

7. The surgical instrument of claim 6, wherein the handle assembly comprises a transducer assembly (112).

8. The surgical instrument of claim 7, wherein the handle assembly comprises a button (5626) configured to activate the transducer.

## Patentansprüche

1. Chirurgisches Instrument (5600), umfassend:
(a) einen Endeffektor (5640), wobei der Endeffektor Folgendes umfasst:
(i) eine Ultraschallklinge (5642) und
(ii) einen Klemmarm (5644), der konfiguriert ist, um relativ zu der Ultraschallklinge zwischen einer offenen Position und einer geschlossenen Position zu schwenken, wobei der Klemmarm einen länglichen Kopplungskörper (5660) umfasst;
(b) eine Wellenanordnung (5630), wobei die Wellenanordnung Folgendes umfasst:
(i) eine Außenhülle (5632) und
(ii) einen Ultraschallwellenleiter (5638), der relativ zu der Außenhülle gesichert ist, wobei der Ultraschallwellenleiter mindestens teilweise innerhalb der Außenhülle untergebracht ist;
(c) einen Schaft (5652), der an dem Klemmarm befestigt ist, wobei der Schaft konfiguriert ist, um relativ zu der Wellenanordnung zu schwenken, um den Klemmarm zwischen der offenen Position und der geschlossenen Position anzutreiben, wobei der Schaft eine Klemmarmstütze (5670) umfasst, die konfiguriert ist, um den länglichen Kopplungskörper des Klemmarms aufzunehmen, um die Befestigung des Klemmarms an dem Schaft zu unterstützen; und
(d) eine Klemmarm-Ausrichtungsstruktur, die konfiguriert ist, um die Ausrichtung des Klemmarms relativ zu der Ultraschallklinge in der geschlossenen Position zu fördern, **dadurch gekennzeichnet, dass** die Klemmarm-Ausrichtungsstruktur eine Schweißplatte (5682) umfasst, die mit der Klemmarmstütze des Schafts assoziiert ist.

2. Chirurgisches Instrument nach Anspruch 1, wobei die Schweißplatte konfiguriert ist, um den länglichen Kopplungskörper des Klemmarms aufzunehmen.

3. Chirurgisches Instrument nach Anspruch 2, wobei die Schweißplatte ein Schweißfenster (5684) derart definiert, dass der längliche Kopplungskörper des Klemmarms konfiguriert ist, um an der Schweißplatte gesichert zu werden, nachdem er zunächst in die Klemmarmstütze eingeführt wurde.

4. Chirurgisches Instrument nach Anspruch 3, wobei der längliche Kopplungskörper konfiguriert ist, um relativ zu der Schweißplatte eingestellt zu werden, bevor er an der Schweißplatte gesichert wird.

5. Chirurgisches Instrument nach Anspruch 4, wobei die Schweißplatte über einen Stift (5656) direkt schwenkbar mit der Außenhülle gekoppelt ist.

6. Chirurgisches Instrument nach Anspruch 1, ferner umfassend eine Griffanordnung (5620), wobei sich die Wellenanordnung von der Griffanordnung distal erstreckt.

7. Chirurgisches Instrument nach Anspruch 6, wobei die Griffanordnung eine Wandleranordnung (112) umfasst.

8. Chirurgisches Instrument nach Anspruch 7, wobei die Griffanordnung einen Schalter (5626) umfasst, der konfiguriert ist, um den Wandler zu aktivieren.

## Revendications

1. Instrument chirurgical (5600), comprenant :
(a) un effecteur terminal (5640), l'effecteur terminal comprenant :
(i) une lame à ultrasons (5642), et
(ii) un bras de serrage (5644) conçu pour pivoter par rapport à la lame ultrasonique entre une position ouverte et une position fermée, dans lequel le bras de serrage comprend un corps d'accouplement allongé (5660) ;
(b) un ensemble arbre (5630), l'ensemble arbre comprenant :
(i) une gaine externe (5632), et
(ii) un guide d'ondes ultrasoniques (5638) fixé par rapport à la gaine extérieure, dans lequel le guide d'ondes ultrasonores est au moins partiellement logé dans la gaine extérieure ;
(c) une tige (5652) fixée au bras de serrage, dans lequel la tige est conçue pour pivoter par rapport à l'ensemble tige afin d'entraîner le bras de serrage entre la position ouverte et la position fermée, dans lequel la tige comprend un support de bras de serrage (5670) conçu pour recevoir le corps d'accouplement allongé du bras de serrage afin de faciliter la fixation du bras de serrage à la tige ; et
(d) une structure d'alignement de bras de serrage conçue pour promouvoir l'alignement du bras de serrage par rapport à la lame ultrasonique en position fermée, **caractérisée en ce que** la structure d'alignement du bras de serrage comprend une plaque de soudage (5682) associée au support de bras de serrage de la tige.

2. Instrument chirurgical selon la revendication 1, dans lequel la plaque de soudage est conçue pour recevoir le corps d'accouplement allongé du bras de serrage.

3. Instrument chirurgical selon la revendication 2, dans lequel la plaque de soudage définit une fenêtre de soudage (5684) de telle sorte que le corps d'accouplement allongé du bras de serrage est conçu pour être fixé à la plaque de soudage après avoir été initialement inséré dans le support de bras de serrage.

4. Instrument chirurgical selon la revendication 3, dans lequel le corps d'accouplement allongé est conçu pour être ajusté par rapport à la plaque de soudage avant d'être fixé à la plaque de soudage.

5. Instrument chirurgical selon la revendication 4, dans lequel la plaque de soudage est directement accouplée de manière pivotante à la gaine extérieure par l'intermédiaire d'une broche (5656).

6. Instrument chirurgical selon la revendication 1, comprenant en outre un ensemble poignée (5620), dans lequel ledit ensemble arbre s'étend à partir dudit ensemble poignée.

7. Instrument chirurgical selon la revendication 6, dans lequel ledit ensemble poignée comprend un ensemble transducteur (112).

8. Instrument chirurgical selon la revendication 7, dans lequel l'ensemble poignée comprend un bouton (5626) configuré pour activer le transducteur.
